# EUROPEAN PATENT SPECIFICATION

(11) **EP 1 677 834 B1**
(45) Date of publication and mention of the grant of the patent: **08.01.2014**
(21) Application number: 04816914.8
(22) Date of filing: 06.10.2004
(51) Int. Cl.: A61K 39/13

(54) **DEFINED DOSE THERAPEUTIC PHAGE**
THERAPEUTISCHER PHAGE MIT DEFINIERTER DOSIS
PHAGE THERAPEUTIQUE DOSE DE MANIERE DEFINIE

(30) Priority: 06.10.2003 US 509308 P
(43) Date of publication of application: 12.07.2006
(73) Proprietor: Gangagen, Inc., Palo Alto, CA 94306 (US)
(72) Inventor: JAYASHEELA, M., Basavanagudi, Bangalore 560004 (IN); SRIRAM, Bharathi, Sanjay Nagar, Bangalore 560094 (IN); PADMANABHAN, Sriram, Sanjay Nagar, Bangalore 560094 (IN)
(74) Representative: Brasnett, Adrian Hugh
(86) International application number: PCT/US2004/033224
(87) International publication number: WO 2005/046579

(56) References cited:
- WO-A-01/50872
- WO-A2-01/79524
- WO-A2-03/080823
- US-A- 4 695 541
- US-A- 6 121 036
- US-A1- 2002 001 590
- LOPEZ R. ET AL.: 'Enzymes for anti-infective therapy phage lysins.' DRUG DISCOVERY TODAY: THERAPEUTIC STRATEGIES. vol. 1, no. 4, December 2004, pages 469 - 474, XP004694307

## Description

### FIELD OF THE INVENTION

The invention provides therapeutic, defined-dose anti-bacterial bacteriophage based preparations, methods to make such preparations, methods to treat bacterial infections using such preparations, methods to diagnose bacterial infections using such preparations, and various host production bacterial strains and related constructs.

### BACKGROUND OF THE INVENTION

Bacteria are ubiquitous, and are found in virtually all habitable environments. They are common and diverse ecologically, and find unusual and common niches for survival. They are present all around the environment, and are present in soil, dust, water, and on virtually all surfaces. Many are normal and beneficial strains, which provide a synergistic relationship with hosts. Others are not so beneficial, or provide problems along with benefits.

Pathogenic bacteria can cause infectious diseases in humans, in other animals, and also in plants. Some bacteria can only make one particular host ill; others cause trouble in a number of hosts, depending on the host specificity of the bacteria. The diseases caused by bacteria are almost as diverse as the bacteria themselves and include food poisoning, tooth ache, anthrax, and even certain forms of cancer. These diseases and the bacteria/host relationships are typically the subject of the field of clinical microbiology.

A variety of "products" of bacterial origin which have lethal effects on some other strains of bacteria have been described. A variety of types of "bacteriocins" are described in literature: some are small molecular weight proteins, which are capable of diffusion; others are coded for by DNA that is present in plasmids; and a third type are high molecular weight (HMW), coded by DNA present in the bacterial genome, and resemble a phage tail.

The HMW bacteriocins are produced by a large number of bacterial species in their natural settings and are thought to play a role in giving the parent bacterium a selective advantage by killing other bacterial strains which may be competing for limited nutrition. These bacteriocins are thermolabile, trypsin resistant, sedimentable by centrifugation, and resolvable by electron microscope. See, e.g., Jabrane, et al. (2002) Appl. Environ. Microbiol. 68:5704-5710; Daw and Falkiner (1996) Micron 27:467-479; Bradley (1967) Bacteriol. Revs 31:230-314; and Kageyama and Egami (1962) Life Sciences 9:471-476.

A tailed bacteriophage generally comprises a head, called the capsid, and a tail. The capsid packages the nucleic acid that is necessary for the further propagation of the bacteriophage in the host bacterium. Therefore, phage tail and phage tail like structures can be similarly described as bacteriophage structures that are essentially devoid of phage DNA. See, e.g., Duckworth (1970) Virology 40:673-684; Chau-te Ou, et al. (1978) Anal. Biochem. 88:357-366. But other artificial assemblies of phage tail components may also retain the critical killing function, while lacking a replicating capacity in a selected target bacterium.

Bacteria are killed in nature by bacteria-specific viruses, e.g., bacteriophage (or phage). Pyocins are believed to be tail-like portions of tailed phages. See, e.g., Abdelhamid, et al. (2002) Appl. Environ. Microbiol. 68:5704-5710; Strauch et al. (2001) Appl. Environ. Microbiol. 67:5635-5642; Nakayama, et al. (2000) Mol. Microbiol. 38:213-31; Daw and Falkiner (1996) Micron 27:467-479; Traub, et al. (1996) Zentralbl. Bakteriol. 284:124-35; Ito, et al. (1986) J. Virol. 59:103-111; Rocourt (1986) Zentralbl. Bakteriol. Mikrobiol. Hyg. 261:12-28; Shinomiya (1984) J. Virol. 49:310-14; and Ishii, et al. (1965) J. Mol. Biol. 13:428-431. However, the relationship of the pyocins and intact phage is not well understood. In particular, it is unclear whether natural isolated pyocins are actually tail portions of derivative bacteriophage, or whether the natural isolated pyocins are further evolved from tail portions.

Certain bacteria are normally innocuous, but become pathogenic upon presentation of the appropriate opportunity, or become problematic upon introduction to an abnormal site or situation. Persons lacking effective immune systems are most vulnerable, and certain bacteria use susceptible weak hosts to provide a temporary environment to proliferate and disperse throughout the ecosystem and a host population.

Statistically, infectious diseases are a major medical problem. See, e.g., Watstein and Jovanovic (2003) Statistical Handbook on Infectious Diseases Greenwood, ISBN: 1573563757. In the U.S., some 40-70K deaths result from bloodstream nosocomial (hospital derived) infections each year.

Synthetic chemical antibiotics have been used to treat bacterial infections for many years, and have minimized the frequency and effects of many infectious diseases. Antibiotics had about $32B worldwide sales in 2002. A great need exists for continued effectiveness of antimicrobial compositions to treat evolving microbial pathogens. The present invention solves these and other problems.

### BRIEF SUMMARY OF THE INVENTION

The present invention provides a composition comprising an anti-bacterial phage-based construct consisting essentially of a phage tail, wherein said phage-based construct is replication incompetent and inhibits growth of a target bacterium. Many problems exist in using replication competent bacteriophage in a therapeutic treatment. For example, the dose changes as the bacteriophage replicate, another is that the replication process inherently allows for the bacteriophage to mutate.

Replication of the DNA or of the anti-bacterial bacteriophage can be prevented by a number of means. One means includes inactivating a nucleic acid of the anti-bacterial phage. Nucleic acid inactivation can be performed by many means, both physically and functionally, e.g. nicking the nucleic acid, fragmenting the nucleic acid, cross-linking said nucleic acid, or by chemically modifying said nucleic acid, or by incorporating misssense, termination, frameshift, conditional, deletion or insertion mutations into critical genes or regulatory elements.

Replication of the anti-bacterial phage can be prevented by removing a nucleic acid from the anti-bacterial phage, in whole or in part. Nucleic acid can be removed by osmotic shock, by a freeze thaw cycle, by chemical methods, or by mechanical methods.

Replication of the anti-bacterial phage can be prevented where the anti-bacterial phage comprises a mutation, deletion or addition, and cannot assemble into a replication competent phage in the target bacterium. In this case the host production bacterium is a complementing host production bacterium that is able to complement the mutation of said anti-bacterial phage and allow replication and production of said anti-bacterial phage in the complementing host production bacterium. In one example, the mutation is a conditional (e.g. temperature sensitive) mutation and the host bacterium complements the mutation at the non-permissive condition (e.g. temperature). In another example a helper phage or expression unit is used to complement the mutation. Means will generally be applied to minimize the possibility of revertant mutations to generate replication competent phage. In other cases, the phage has incorporated a function which prevents replication, e.g. a restriction site or enzyme which leads to degrading the phage DNA; the phage may incorporate a gene expressed early which prevents DNA or phage replication; or the phage may contain a deletion of a critical replication function.

Also described is a complementing host bacterium and complementing helper phage for use in production of the anti-bacterial phage.

In one aspect of the invention the anti-bacterial phage has no detectable replication activity in the target bacterium. In another aspect the anti-bacterial phage kills the host bacterium.

As herein described, the anti-bacterial phage has less than 98% of the complexity of DNA of an intact parental phage; less than 20% or 2% of the a nucleic acid content of an intact parental phage; does not contain detectable nucleic acid; is an intact phage having nucleic acid with reduced (e.g. by 10% or more) replication capacity; comprises a tail portion of a tailed phage or comprises an electron microscope morphologically identifiable tail portion of a tailed phage.

In various other embodiments, the pharmaceutical composition further includes a therapeutically compatible buffer or excipient, or includes a second therapeutic or anti-microbial agent. The second therapeutic agent may be, e.g. an inflammatory agent, or the second microbial agent can be, e.g., an antibiotic or a second anti-bacterial phage.

Also described are methods of making an anti-bacterial phage or fragment thereof. An anti-bacterial phage can be made, e.g. (1) by amplifying a phage in a host bacterium, harvesting the phage from the bacterial culture, and removing substantially all of the nucleic acids from the phage; (2) by amplifying a phage in a host bacterium, harvesting the phage from the bacterial culture, and inactivating the nucleic acids of the phage; (3) by amplifying a phage in a host bacterium, harvesting the phage from the bacterial culture substantially before intact phage are produced; or (4) by amplifying a phage in a host bacterium, harvesting the phage from the bacterial culture, and harvesting the phage from the bacterial culture, wherein an nucleic acid of said anti-bacterial phage comprises a mutation and cannot assemble into a replication competent phage, and wherein the host bacterium is a complementing host production bacterium that is able to complement the mutation of said anti-bacterial phage and allow replication of said anti-bacterial phage in the complementing host production bacterium.

The present invention provides a composition as herein defined for use in a method of treating a bacterial population of infection in a subject by administering a therapeutically or prophylactically effective amount of a pharmaceutical compound including an anti-bacterial phage or anti-bacterial phage fragments. In one embodiment, the bacterial infection is caused by the target bacterium, e.g. E.coli. The subject of treatment can be a human, a primate, a food, working, companion or display animal. A second therapeutic agent may be administered, e.g. an anti-microbial agent, an antibiotic, or a second anti-bacterial phage. The pharmaceutical composition can be administered systemically, e.g. parenterally or orally, locally, e.g. topically or by inhalation, or otherwise, including by catheter or drain tube; and may result in a relative decrease in the target population of at least 10-1000 fold, or a decrease in detectability by at least 5-50 fold.

The present invention provides a pharmaceutical composition including a genetically incompetent anti-bacterial phage, e.g. that inhibits growth of a target bacterium. In various embodiments of the present invention, the target bacterium is identified or diagnosed, including Escherichia, Staphylococcus, Pseudomonas or Streptococcus. As described the genetically incompetent anti-bacterial phage may lack a full complement of genetic material, including deletions from a full complement; have a mutation and cannot or can only slowly assemble into a replication competent phage in the target bacterium, e.g. a defective critical structural component; inappropriate stoichiometry of components, or a defective critical assembly component; comprise nucleic acid with a reduced replication capacity, e.g. contain a mutation (missense, termination, frameshift, conditional, deletion or insertion) in a critical phage replication function. In one embodiment the phage consists essentially of a tail protein from a tailed phage, including a myoviridae or syphoviridae phage. Also described are methods for making such a composit8ion or formulating a pharmaceutical composition, e.g. with an excipient, buffer or other therapeutic.

The present invention also provides a method of using a pharmaceutical composition including a genetically incompetent anti-bacterial phage or fragment to treat a bacterial population or infection, e.g. by administering an effective amount of the pharmaceutical composition including the genetically incompetent anti-bacterial phage. The use can be in the treatment of a human, a primate, a food, work, companion or display animal. The pharmaceutical can be administered systemically, e.g. parenterally or orally, locally, e.g. topically or by inhalation; or by other methods. A second therapeutic or prophylactic agent, e.g. antimicrobial agent, antibiotic or a second anti-bacterial phage, can be administered with the anti-bacterial phage composition.

Also described is a method of identifying an anti-bacterial phage that is unable to replicate in a target bacterium by identifying a target bacterium, identifying a phage (e.g. natural isolates or selected mutationally diversified populations) that can inhibit growth of the target bacterium, and determining or generating a form of the phage (e.g. a fragment thereof) that is unable to replicate in the target bacterium. Also described are anti-bacterial phages that have been identified using this method. Once identified, the phages may be further isolated, characterised and modified.

Also described is a method of producing an anti-bacterial phage by amplifying a phage, e.g. an intact parental phage, in a host bacterium, harvesting the phage from the bacterial culture, and removing substantially all of the nucleic acids from the phage. The phage may be a tailed phage; and the nucleic acids can be removed by a variety of methods including, e.g. osmotic shock, freeze thaw cycle, chemical methods or mechanical methods; fragmenting the phage into binding specificity components (tails) separate from DNA containing packets (heads) and isolating the tails separately from the heads; separating a tail from a head of the tailed phage, and isolating the tail; harvesting the phage before a head and a tail have assembled to form an intact phage, and isolating the tails; and genetically mutating the phage so they cannot produce or package the nucleic acids, e.g. with a missense, termination, frameshift, conditional, deletion or insertion mutation. Additional means to remove remaining intact phage heads or residual DNA may be included, e.g. sedimentation methods, affinity reagents, DNA degradation methods, etc.

Also described are methods of making a defined dose anti-bacterial phage that kills a target bacterium, by producing said phage in: a host production bacterium and isolating tail portions from DNA containing heads; a host production bacterium and inactivating nucleic acid of said phage, e.g. buy nicking, fragmenting, crosslinking, chemically modifying or removing; a host production bacterium and harvesting temporally before phage assembly; a complementing host; capable of complementing a blockage of replication in the target, including with use of a helper phage; or a permissive host which phage are non-permissive in a different condition, e.g. temperature. The method can be performed using a tailed phage, including a myoviridae or syphoviridae, and, e.g. separating the anti-bacterial phage tails from DNA containing heads.

The anti-bacterial phage can: include a replication blocking mutation, e.g. a point mutation; missense, termination, frameshift, conditional, deletion or insertion; be produced in a complementing host production bacterium, with or without a helper phage or the like; exhibit less than 99% of the complexity of a replication capable parental phage; exhibit less than 20%, or 5% of the DNA or phage replication activity in the target bacterium compared to the host production bacterium; exhibit diminished immunogenicity compared to intact parental phage upon presentation to a mammal, e.g. by at least about 30%, 60%, 90%, 95%, 99% in total immune response or number of epitopes responded to; exhibit little or no significant DNA replication or phage replication capacity in the target bacterium; kill a pathogenic, nocosomial, pyogenic, Gram negative, Gram positive, Escherichia, Staphylococcus, Pseudomonas or Streptococcus bacterium; or kill a food or environmental contaminating bacterium. Also described are the host production bacteria and helper phage, for production or pharmaceutical compositions containing anti-bacterial phage.

Where the anti-bacterial phage is produced in a complementing host, the anti-bacterial phage can include any of the following replication blocking mutations: a point mutation; a deletion mutation; or an insertion mutation in a gene necessary for replication in said target bacterium. The complementing host can be selected to provide the function of the mutated gene product and described is the complementing host bacterium. Described are methods to produce defined dose anti-bacterial phage that exhibit diminished capacity to transmit toxin genes in the target bacteria when compared to intact phage in the host bacterium, as well as defined dose anti-bacterial phage that have diminished immunogenicity, e.g. encoding fewer epitopes, upon administration to a mammal as compared to intact phage from a host production bacteria.

Described are defined dose anti-bacterial phage that exhibit lesser, or no detectable replication activity in the target bacterium.

The defined dose anti-bacterial phage can be used to treat bacteria, e.g. a pathogenic bacterium, such as a nocosomial or pyogenic bacterium. In one embodiment, the pathogenic bacterium is a Gram negative bacterium, e.g. an E.coli bacterium.

Described is a defined dose anti-bacterial composition comprising a phage protein derived from an intact parental phage or prophage, where the anti-bacterial composition is capable of killing a target bacterium, and the composition exhibits less than about 20% or 5% replication activity in the target bacterium as compared to the intact parental phage. In various embodiments, the anti-bacterial phage exhibits: diminished capacity to transmit toxin genes in the target bacteria when compared to intact phage in the host bacterium; diminished immunogenicity as compared to intact phage from a host production bacteria upon administration to a mammal; or no substantial, e.g. less than about 20%, replication activity in the target bacterium.

The defined dose anti-bacterial composition can be used to kill a target bacterium that is pathogenic, nosocomial, pyogenic, Gram negative, environmental, or food bacteria, including an Escherichia, Staphylococcus, Pseudomonas, or Streptococcus bacterium.

The defined dose anti-bacterial composition comprising a phage protein can also include a nucleic acid with reduced nucleic acid content or replication capacity, e.g. nicked, fragmented, cross linked or UV irradiated. The defined dose anti-bacterial composition, comprising a phage protein can also possess less than 20% of the nucleic acid content of the intact parental phage; can lack any detectable nucleic acid; or can include damaged DNA that is unable to be replicated.

The defined dose anti-bacterial composition comprising a phage protein can be a tail portion derived from an intact parental phage that is a tailed phage. Methods for screening variants of natural bacteriophage for specific isolates or desired properties and conversion of such isolates into replication deficient forms for therapy are provided herein.

The defined dose anti-bacterial composition comprising a phage protein can include a second agent, including e.g. another therapeutic or prophylactic compound, e.g. an inflammatory agent, anti-microbial, antibiotic, bacterial cell wall growth disrupting compound, or a second anti-bacterial phage. The defined dose anti-bacterial composition can also include a therapeutically compatible buffer or excipient.

The defined dose anti-bacterial composition comprising a phage protein can be made: by processing intact parental phage to remove or inactivate nucleic acids; or by harvesting phage from a host bacterium before intact phage are assembled; or in a complementing host strain, where the parental strain is defective in expressing critical genes for assembly, production, release, or infection by said phage. The defect can be a result of a point mutation, including a missense, termination, frameshift, conditional, deletion or insertion that prevents phage replication or production.

The defined dose anti-bacterial composition comprising a phage protein can be administered to a eukaryote suffering from a bacterial infection or colonization by the target bacterium. The eukaryote can be a mammal, including a primate, and may be a food, work, display or companion animal.

The target bacterium can be a pathogenic, nosocomial, or pyogenic bacterium. In one embodiment, the target bacterium is E.coli. In one embodiment, the infection has been diagnosed to be susceptible to the composition.

The defined dose anti-bacterial composition comprising a phage protein can be administered systemically, locally, parentally, orally, topically, by inhalation, catheter, or drip tube; or with an antibiotic, anti-microbial, or other therapeutic or prophylactic agent. In one embodiment, the infection has already been treated with an antibiotic. The eukaryote can also be inoculated with another bacterium to replace the target bacterium.

The present invention provides a therapeutic anti-bacterial composition as herein defined. The composition can be used therapeutically or prophylactically to treat a food, work, display or companion animal, or primate. The target bacterium can be a pathogenic bacterium. The composition can be administered topically or systemically. The composition can be administered in combination with a second compound, such as an anti-bacterial agent, antibiotic, DNA replication inhibitors, protein, lipid or cell wall growth inhibitors, inflammatory agent, or excipient.

### BRIEF DESCRIPTION OF THE DRAWINGS

### I. INTRODUCTION

The present invention provides anti-bacterial phages or parts thereof that are unable to replicate in a target bacteria and that also inhibit growth of the target bacteria. The anti-bacterial phages are thus useful for inhibiting bacterial growth or presence in the environment and for treating bacterial infection in a subject in need of such treatment. Because the anti-bacterial phage compositions are unable to replicate in a target bacteria, they can be administered as a defined dose therapeutic composition for treatment of bacterial infections. This provides substantial regulatory advantages, which prevent changing stoichiometric ratios of treatment and target entities as the bacterial infection and bacteriophage replication processes progress.

This invention provides the first disclosure that, for each pathogenic bacteria target, a tailed portion of a phage from the Siphoviridae or Myoviridae families will be useful as a defined dose therapeutic agent to inhibit growth of or kill the pathogenic bacteria. The relationship of natural pyocins to phage tail portions has not been established. The proposition that tails can be isolated from most any bacteriophage exhibiting a desired host range and thereafter converted into defined dose compositions greatly enlarges the universe of potential sources and the means to isolate desired tail fragments.

### II. DEFINITIONS

As used herein "bacteriophage" is generally shortened to "phage". Bacteriophage typically refers to a functional phage, but in many contexts herein may refer to a part thereof, generally exhibiting a particular function. The phage may be lytic or lysogenic. See, e.g., Chen and Lu (2002) Applied and Env. Microbiol. 68:2589-2594. In some circumstances, the term may also refer to portions thereof, including, e.g., a tail portion, a head portion, or an assembly of components which provide substantially the same functional activity. The portion may be a physical fragment of an intact phage, a selected product from normal or abnormal assembly of phage parts, or even an artificial construct, e.g., from genetic manipulation of genes encoding (1) phage parts, (2) critical phage assembly components, or even (3) associated host genes which may be useful in ensuring phage replication or production. When referring to a phage genome, typically the term refers to a naturally occurring phage genome, but may include fragments, artificial constructs, mutagenized genomes, selected genomes, and particularly "prophage" sequences, which are considered to be "defective" genomes which may have had segments deleted, inserted, or otherwise affected to disrupt normal genome function.

Typically, phage will be morphologically identifiable, having a size which is resolvable by imaging methods, e.g., electron microscopy. See, e.g., Ackermann and Nguyen (1983) Appl. Environ. Microbiol. 45:1049-1059; Tsaneva (1976) Appl. Environ. Microbiol. 31:590-601; Talledo, et al. (2003) Envision. Microbiol. 5:350-354; and Duda and Eiserling (1982) J. Virol. 43:714-720.

An "anti-bacterial phage" is a phage or phage-based construct (e.g., a phage tail, tail fragment, phage protein, or ghost phage) that is unable to replicate, DNA or the phage itself, or assemble in a target bacterium, but that inhibits the growth, survival, or replication of the target bacterium. Thus, an "anti-bacterial phage" can include a portion of a phage that can be used to inhibit growth of the target bacterium and lacks capacity to replicate itself in the target. For example, an antibacterial phage can be a portion of an intact phage that can be produced in a non-target bacteria. Thus, as defined herein, an anti-bacterial phage can include a structural portion of an intact phage, e.g., a tail portion of a tailed phage; or an isolated protein component of an intact phage. These phage-based compositions include one or more proteins or protein domains derived from a natural or engineered bacteriophage.

Certain embodiments of anti-bacterial phage include constructs which contain less than about 70%, 50%, 20%, 5%, 2%, 1 %, 0.1 %, or less of the parental phage nucleic acid content. The content may be either mass, or informational content, e.g., where some portion of the informational content is deleted.

Those of skill will recognize that phage are viruses that infect bacteria. Anti-bacterial phages include a phage from the families Podoviridae, Siphoviridae, Myoviridae, Lipothrixviridae, Plasmaviridae, Corticoviridae, Fuselloviridae, Tectiviridae, Cystoviridae, Levividae, Microviridae, Inoviridae plectrovirus, and Inoviridae inovirus. See Ackermann and Dubow (1987) Viruses of Prokaryotes CRC Press, ISBN: 0849360544). In some embodiments the antibacterial phage is derived from a tailed phage from the families Podoviridae, Siphoviridae, and Myoviridae. In a typical embodiment the anti-bacterial phage is derived, e.g., by mutagenesis or engineered, from a naturally occurring or wild-type tailed phage from the family myoviridae or from the family Siphoviridae.

As used herein, "target bacterium" or "target bacteria" refer to a bacterium or bacteria whose growth, survival, or replication is inhibited by an antibacterial phage. "Growth inhibition" can refer, e.g., to slowing of the rate of bacterial cell division, or cessation of bacterial cell division, or to death of the bacteria. In a typical embodiment, the "target bacterium" or "target bacteria" are pathogenic bacteria.

As used herein, "host bacterium" or "host bacteria" refer to a bacterium or bacteria used to produce, replicate, or amplify a phage, sometimes referred to as a parental phage, that is used to produce an anti-bacterial phage. Host bacteria or bacterium are also referred to as "host production bacterium" or "host production bacteria," throughout. In one embodiment, the parental phage is a prophage, e.g., a defective or incomplete phage genome. Often the host production culture complements a defect in the phage, or suppresses a destructive function encoded in the phage. In other embodiments, the host production culture may make use of a helper phage to effect the capability.

An anti-bacterial phage is a phage that, in addition to its growth inhibitory activity, is essentially unable to replicate in the target bacterium under the conditions of use. As used herein, "replication" refers to phage nucleic acid replication, or to production of a phage. As used herein, "replication" or "replication activity" in the context of nucleic acids refers to replication of genetic material, e.g., DNA or RNA. Replication can also refer to replication of a functional phage, which may involve assembly of an intact phage, and includes synthesis of components of the phage, including proteins; and assembly of the components of the phage to form an intact phage. Components of the phage include, e.g., tails, heads, or nucleic acids. Replication typically leads to the production of "an intact phage," which is a phage that is able to replicate itself in a non-target bacteria. Thus, a replication deficient phage is a phage that is deficient in one or more of the processes noted above. Standard methods are conveniently used to evaluate the replication capacity of a construct. For example, the ability to form plaques on a host bacterial lawn can be used. Typically, inactivation will decrease function, e.g., the replication capacity by at least 3 fold, and may affect it by 10, 30, 100, 300, etc., to many orders of magnitude.

Loss of replication activity by an anti-bacterial phage, (also referred to as being unable to replicate, loss of assembly activity, and genetically incompetent anti-bacterial phage), can occur, e.g., through removal of all or critical portions of nucleic acids, inactivation of nucleic acids, removal of structural portions of a phage, e.g., removal of the head of a tailed phage. The replication activity of an anti-bacterial phage in a target bacterium is preferably measured relative to the replication activity of the parental phage in the host bacterium, or relative the parental phage in the target bacterium. Thus, an anti-bacterial phage can exhibit less than 10%, 1%, 0.1 %, 0.01 %, 0.001% or 0.0001 % of the levels of nucleic acid, e.g., DNA or RNA, or polymerase activity of a parental phage. Diminished polymerase activity can occur because of changes in the enzyme or changes in the substrate nucleic acids, e.g., removal or inactivation of the nucleic acid of an anti-bacterial phage. In another embodiment the anti-bacterial phage, can have less than 10%, 1%, 0.1%, 0.01%, 0.001% or 0.0001% of the levels of a component of the parental phage, e.g., DNA or RNA, phage heads, or specific phage proteins. Inactive phage may result from aberrant stoichiometric ratios of structural or functional components.

Anti-bacterial phage can also include phage whose nucleic acids have been inactivated or functionally modified. Those of skill will recognize that many methods can be used to inactivate nucleic acids, e.g., UV and X ray irradiation, fragmentation of DNA, and/or treatment with chemicals including D-glucosamine and ferrous ammonium sulfate.

Anti-bacterial phage also include phage constructs whose nucleic acid has been partially or totally removed. Some such phage are also referred to as "ghosts" or "ghost phage." Methods to remove nucleic acids from phage and make anti-bacterial phage include removal of all or substantially all of the structural components that contain phage nucleic acids, e.g., retaining the tails of a tailed phage. Nucleic acid can also be removed by compromising the structural integrity of a phage, e.g. by osmotic shock with a salt or sugar; freezing and thawing the phage; and chemical treatments, including treatment with the following: LiCl or other salts, EDTA or other chelating agents, organic salts, amino acids, and reducing agents; and mechanical methods including the following: shearing, lyophilization, sonication, and microwave treatment.

Anti-bacterial phage also include phage that comprise a mutation and cannot efficiently assemble into a replication competent phage in the target bacteria. Mutations can include mutations in genes that encode enzymes for replication of nucleic acids or genes that encode regulators of replication; or in genes that encode structural components of a phage or genes that encode regulators of the synthesis of structural components, or genes that encode proteins critical for assembly, e.g., assembly functions, or genes that regulate stoichiometry of proteins necessary for proper assembly. The mutations can be in the coding region of a gene or in a regulatory region of the gene, e.g., a promoter.

Such an anti-bacterial phage will typically be produced in a "complementing host production bacterium." A complementing host production bacterium comprises a complementing nucleic acid or activity, e.g., in a plasmid or supplied by a helper phage, that complements the mutation comprised by the anti-bacterial phage. In some embodiments, the bacterium comprises a nucleic acid that encodes a protein that supplies the function of the mutated protein in the anti-bacterial phage. The complementing nucleic acid can be part of the bacterial genome or part of an extra-genomic element, e.g., a plasmid. In one embodiment, a second phage in the bacterium comprises the complementing nucleic acid, e.g., a helper phage. Examples of phage mutations and complementing host or phage include a) phage comprising termination mutations and complementing host or phage comprising tRNA suppressors, b) phage comprising mutations in genes critical for replication, production, or assembly, and complementing host or phage comprising antisense constructs that complement the mutation, c) phage comprising insertion mutations and complementing host or phage that comprise suppressors of the mutations, d) phage comprising deletion mutations and complementing host or phage that comprise suppressors of the mutations, and e) phage which encode an additional deleterious function, e.g., a restriction or phage exclusion system, and complementing host or phage that comprise an inactivating function, e.g., a modification system. See, e.g., on restriction-modification systems: King and Murray (1994) Trends Microbiol. 2:465-69; Bickle (2004) Molec. Microbiol. 51:3-5; Kobayashi, et al. (1999) Curr. Op. Genetics Dev. 9:649-56; and Catalano (1994) Medicina (B. Aires) 54:596-604; and on phage-exclusion: Pecota and Wood (1996) J. Bacteriol. 178:2044-2050.

An "anti-microbial agent" is an agent or compound that can be used to inhibit the growth of or to kill bacteria. Anti-microbial agents include antibiotics, chemotherapeutic agents, antibodies (with or without complement), chemical inhibitors of DNA, RNA, protein, lipid, or cell wall synthesis or functions, and anti-bacterial phages, usually referring to the second or more phages when more than one anti-bacterial phage is present in a compound or used in a method of the present invention.

As used herein, "amplifying a phage in a host bacterium" refers to infecting a host bacterium with a parental phage under conditions that allow the DNA or phage to replicate and make copies of itself. As used herein, "harvesting a phage from a bacterial culture" refers to removing the phage from the host bacterial culture. In some embodiments, the phage can have the attributes of an anti-bacterial phage, e.g., ability to inhibit growth of the target bacterium and inability to replicate in the target bacterium. In other embodiments, the phage can be treated, before or after removal from the bacterial culture, to produce an anti-bacterial phage, e.g., through removal or inactivation of nucleic acids. In a further embodiment, the anti-bacterial phage can be further purified to remove residual replication competent phage before application to the target bacteria, e.g., administration to a subject infected with the target bacteria.

A "bacterial infection" refers to growth of bacteria, e.g., in a subject or environment, such that the bacteria actually or potentially could cause disease or a symptom in the subject or environment. This may include prophylactic treatment of substances or materials, including organ donations, medical equipment such as a respirator or dialysis machine, or wounds, e.g., during or after surgery, e.g., to remove target bacteria which may cause problems upon further growth

A "subject in need of treatment" is a animal or plant with a bacterial infection that is potentially life-threatening or that impairs health or shortens the lifespan of the animal. The animal can be a fish, bird, or mammal. Exemplary mammals include humans, domesticated animals (e.g., cows, horses, sheep, pigs, dogs, and cats), and exhibition animals, e.g., in a zoo. In some embodiments anti-bacterial phage are used to treat plants with bacterial infections, or to treat environmental occurrences of the target bacteria, such as in a hospital or commercial setting.

A "pharmaceutically acceptable" component is one that is suitable for use with humans, animals, and/or plants without undue adverse side effects (such as toxicity, irritation, and allergic response) commensurate with a reasonable benefit/risk ratio.

A "safe and effective amount" refers to a quantity of a component that is sufficient to yield a desired therapeutic response without undue adverse side effects (such as toxicity, irritation, or allergic response) commensurate with a reasonable benefit/risk ratio when used in the manner of this invention. By "therapeutically effective amount" is meant an amount of a component effective to yield a desired therapeutic response, e.g., an amount effective to slow the rate of bacterial cell division, or to cause cessation of bacterial cell division, or to cause death or decrease rate of population growth of the bacteria. The specific safe and effective amount or therapeutically effective amount will vary with such factors as the particular condition being treated, the physical condition of the subject, the type of subject being treated, the duration of the treatment, the nature of concurrent therapy (if any), and the specific formulations employed and the structure of the compounds or its derivatives.

"Nucleic acid" refers to deoxyribonucleotides or ribonucleotides and polymers (pure or mixed) thereof in single- or double-stranded form. The term encompasses nucleic acids containing nucleotide analogs or modified backbone residues or linkages, which are synthetic, naturally occurring, and non-naturally occurring, which have similar binding, structural, or functional properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, and peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues. See, e.g., Batzer, et al. (1991) Nucleic Acid Res. 19:5081-xxxx; Ohtsuka, et al. (1985) J. Biol. Chem. 260:2605-2608; Rossolini, et al. (1994) Mol. Cell. Probes 8:91-98. The term nucleic acid is typically used interchangeably with gene, cDNA, mRNA, oligonucleotide, and polynucleotide.

A particular nucleic acid sequence also implicitly encompasses "splice variants." Similarly, a particular protein encoded by a nucleic acid implicitly encompasses a protein encoded by a splice variant of that nucleic acid. "Splice variants," as the name suggests, are products of alternative splicing of a gene segment. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Products of a splicing reaction, including recombinant forms of the splice products, are included in this definition.

The terms "polypeptide," "peptide," and "protein" are typically used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refer to compounds that have a similar basic chemical structure or function as a naturally occurring amino acid, e.g., an α carbon that is bound to a hydrogen, a carboxyl group, an amino group, and an R group, e.g., homoserine, norleucine, methionine sulfoxide, and methionine methyl sulfonium. Such analogs have modified R groups (e.g., norleucine) or modified peptide backbones, but retain a similar basic chemical structure as a naturally occurring amino acid. Amino acid mimetic refers to a chemical compound that has a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to sequences exhibiting essentially identical function. Because of the degeneracy of the genetic code, a large number of functionally identical nucleic acids can encode a given protein. For instance, the codons GCA, GCC, GCG, and GCU all encode the amino acid alanine. Thus, at every position where an alanine is specified by a codon, the codon can be altered to the corresponding codons described without altering the encoded polypeptide. Such nucleic acid variations are typically "silent variations," which are one species of conservatively modified variations. Every nucleic acid sequence herein which encodes a polypeptide also describes every silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are typically considered conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton (1984) Proteins*).*

Macromolecular structures such as polypeptide structures can be described in terms of various levels of organization. For a general discussion of this organization, see, e.g., Alberts, et al. (1994) Molecular Biology of the Cell (3d ed.) and Cantor and Schimmel (1980) Biophysical Chemistry Part I: The Conformation of Biological Macromolecules*.* "Primary structure" refers to the amino acid sequence of a particular peptide. "Secondary structure" refers to locally ordered, three dimensional structures within a polypeptide. These structures are commonly known as domains, e.g., transmembrane domains, pore domains, and cytoplasmic tail domains. Domains are generally portions of a polypeptide that form a compact unit of the polypeptide and are typically 15 to 350 amino acids long. Exemplary domains include domains with enzymatic activity, e.g., phosphatase domains, ligand binding domains, etc. Typical domains are made up of sections of lesser organization such as stretches of β-sheet and α-helices. "Tertiary structure" refers to the complete three dimensional structure of a polypeptide monomer. "Quaternary structure" refers to the three dimensional structure formed typically by noncovalent association of independent tertiary units. Anisotropic terms are also known as energy terms.

A "label" or a "detectable moiety" is a composition detectable by spectroscopic, photochemical, biochemical, immunochemical, chemical, or other physical means. For example, useful labels include 32P, fluorescent dyes, electron dense reagents, enzymes (e.g., as commonly used in an ELISA), biotin, digoxigenin, or haptens and proteins which can be made detectable, e.g., by incorporating a radiolabel into the peptide or used to detect antibodies specifically reactive with the peptide.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed, or not expressed at all.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. See, e.g., Paul (2003) Fundamental Immunology (5th ed.) Lippincott.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains, respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (see Paul (1993) Fundamental Immunology (3d ed.). While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries (see, e.g., McCafferty, et al. (1990) Nature 348:552-554).

For preparation of antibodies, e.g., recombinant, monoclonal, or polyclonal antibodies, many techniques known in the art can be used (see, e.g., Kohler & Milstein (1975) Nature 256:495-497; Kozbor, et al. (1983) Immunology Today 4:72-xx; Cole, et al. (1985) pp. 77-96 in Monoclonal Antibodies and Cancer Therapy, Alan R. Liss, Inc.; Coligan (1991) Current Protocols in Immunology*;* Harlow and Lane (1988) Antibodies, A Laboratory Manual*;* and Goding (1986) Monoclonal Antibodies: Principles and Practice (2d ed.)). Genes encoding heavy and light chains of an antibody of interest can be cloned from a cell, e.g., the genes encoding a monoclonal antibody can be cloned from a hybridoma and used to produce a recombinant monoclonal antibody. Gene libraries encoding heavy and light chains of monoclonal antibodies can also be made from hybridoma or plasma cells. Random combinations of the heavy and light chain gene products generate a large pool of antibodies with different antigenic specificity (see, e.g., Kuby (1997) Immunology (3d ed.)). Techniques for the production of single chain antibodies or recombinant antibodies (U.S. Patent 4,946,778, U.S. Patent No. 4,816,567) can be adapted to produce antibodies to polypeptides of this invention. Also, transgenic mice, or other organisms such as other mammals, may be used to express humanized or human antibodies (see, e.g., U.S. Patent Nos. 5,545,807; 5,545,806; 5,569,825; 5,625,126; 5,633,425; 5,661,016, Marks, et al. (1992) Bio/Technology 10:779-783; Lonberg, et al. (1994) Nature 368:856-859; Morrison (1994) Nature 368:812-13; Fishwild, et al. (1996) Nature Biotechnology 14:845-51; Neuberger (1996) Nature Biotechnology 14:826-xx; and Lonberg and Huszar (1995) Intern'l. Rev. Immunol. 13:65-93). Alternatively, phage display technology can be used to identify antibodies and heteromeric Fab fragments that specifically bind to selected antigens (see, e.g., McCafferty, et al. (1990) Nature 348:552-554; Marks, et al. (1992) Biotechnology 10:779-783). Antibodies can also be made bispecific, e.g., able to recognize two different antigens (see, e.g., WO 93/08829, Traunecker, et al. (1991) EMBO J. 10:3655-3659; and Suresh, et al. (1986) Methods in Enzymology 121:210). Antibodies can also be heteroconjugates, e.g., two covalently joined antibodies, or immunotoxins (see, e.g., U.S. Patent No. 4,676,980 , WO91/00360; WO92/200373; and EP03089)

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

In one embodiment, the antibody or phage tail is conjugated to an "effector" moiety. The effector moiety can be any from a number of molecules, including labeling moieties such as radioactive labels or fluorescent labels, or can be a therapeutic moiety. Some therapeutic moieties may provide high enzymatic turnover, providing large activities per moiety, and my be important in attracting or inducing natural physiological reactions which may assist in the desired therapeutic result, e.g., attracting macrophages or other components of the immune system.. In one aspect the antibody modulates the activity of the protein.

The phrase "specifically (or selectively) binds" to an antibody or "specifically (or selectively) immunoreactive with," when referring to a protein or peptide or anti-bacterial phage comprising a protein, refers to a binding reaction that is determinative of the presence of the protein, often in a heterogeneous population of proteins and other biologics. Thus, under designated immunoassay conditions, the specified antibodies bind to a particular protein at least two times the background and more typically more than 10 to 100 times background. Specific binding to an antibody under such conditions requires an antibody that is selected for its specificity for a particular protein. This selection may be achieved by subtracting out (depleting, e.g., by absorption) antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (see, e.g., Harlow and Lane (1988) Antibodies, A Laboratory Manual for a description of immunoassay formats and conditions that can be used to determine specific immunoreactivity).

### III. PATHOGENIC BACTERIA

In a preferred embodiment, the anti-bacterial phage are used to inhibit growth, survival, or replication of a desired, e.g., pathogenic, bacteria. The bacteria may be an environmentally deleterious strain, or may be treated in a prophylactic manner.

### A. Natural, infective, pathogenic bacteria

In a healthy animal, the internal tissues, e.g. blood, brain, muscle, etc., are normally free of microorganisms, particularly bacteria. On the other hand, the surface tissues, e.g., skin and mucous membranes, are constantly in contact with environmental organisms and become readily colonized by certain microbial species. The normal flora is the mixture of organisms regularly found at an anatomical site, e.g., skin, conjunctiva, nose, pharynx, mouth, lower intestine, anterior urethra, and vagina.

The normal flora of humans, e.g., is exceedingly complex and consists of more than 200 species of bacteria. Clinical microbiology studies these and pathogenic strains, and other aspects of the related biology relevant to human health. See, e.g., Sarma (2001) Medical Microbiology: A Clinical Perspective Paras, Heyderabad, ISBN: 8188129070; Baron, et al. (1994) Bailey & Scott's Diagnostic Microbiology (9th ed.), ISBN: 0801669871; Balows, et al. (eds. 1991) Manual of Clinical Microbiology (5th ed.) Am. Soc. Microbiol., Wash. D.C., ASIN: 1555810306; Hobbs, et al. (1991) Medical Microbiology for Students Arnold, New Delhi; and Fessia, et al. (1988) Diagnostic Clinical Microbiology: A Benchtop Perspective Saunders, Philadelphia, ISBN: 0721623263. The makeup of the normal flora depends upon various factors, including genetics, age, sex, stress, nutrition, and diet of the individual. The normal flora of humans includes eukaryotic fungi and protists, and some methanogenic Archaea that colonize the lower intestinal tract, but bacteria are the most numerous and obvious microbial components of the normal flora.

The normal flora are typically adapted to their host (tissues), most probably by biochemical interactions between bacterial surface components (ligands or adhesins) and host cell molecular receptors. Much information is available on the nature of adhesion of bacterial pathogens to animal cells and tissues, and reasonably similar mechanisms should apply to the normal flora.

Little is known about the nature of the associations between humans and their normal flora, but they are thought to be dynamic interactions rather than associations of mutual indifference. Both host and bacteria are thought to derive benefit from each other, and the associations are, for the most part, mutualistic. The normal flora derives from the host a supply of nutrients, a stable environment, and constant temperature, protection, and transport. The host obtains from the normal flora certain nutritional benefits, stimulation of the immune system, and colonization strategies that exclude potential pathogens at the site.

A pathogenic microorganism generally causes disease, sometimes only in particular circumstances. Pathogenicity is the ability to produce disease or deleterious symptoms in a host organism. Microbes express their pathogenicity by means of their virulence, a term which refers to the degree of pathogenicity of the microbe. Hence, determinants of virulence of a pathogen are its genetic or biochemical or structural features that enable it to produce disease or symptoms in a host.

The relationship between a host and a pathogen is dynamic, since each modifies the activities and functions of the other. The outcome of an infection depends on the virulence of the pathogen and the relative degree of resistance or susceptibility of the host, due mainly to the effectiveness of the host defense mechanisms.

Historically, bacteria have been the cause of some of the most deadly diseases and widespread epidemics of human civilization. See, e.g., Cohen (2003) Infectious Diseases, Elsevier, ISBN: 0323024076; Gorbach, et al. (eds. 2003) Infectious Diseases Lippincott Williams & Wilkins, ISBN: 0781733715; Turkington, et al. (2003) Encyclopedic of Infectious Diseases (2d ed.) Facts on File, ISBN: 0816047758; Watstein and Jovanovic (2003) Statistical Handbook on Infectious Diseases Greenwood, ISBN: 1573563757; Mandell (2000) Principles and Practice of Infectious Diseases Elsevier, ISBN: 0443065810; Gorbach and Stone (2000) Atlas of Infectious Diseases, Harcourt, ISBN: 0721670326; Root, et al. (eds. 2000) Clinical Infectious Diseases: A Practical Approach, Oxford University Press, ISBN: 0195143493; Schlossberg (2000) Current Therapy of Infectious Disease (2d ed.) Elsevier, ISBN: 0323009077; and Mandell, et al. (1998) Mandell, Douglas and Bennett's Principles and Practice of Infectious Diseases, Churchill Livingstone, ISBN: 044307593X.

Public health measures, e.g., water purification, immunization, and modem antibiotic treatment, have reduced the morbidity and the mortality of bacterial disease in the Twentieth Century, at least in the developed world where these are acceptable cultural practices. However, many new bacterial pathogens have been recognized in the past 25 years and many "old" bacterial pathogens, such as Staphylococcus aureus and Mycobacterium tuberculosis, have emerged with new determinants of virulence as well as new patterns of resistance to antimicrobial agents.

### B. Nosocomial (hospital derived) infections, environmental bacteria, and pyogenic (pus forming) bacteria

The methods and compositions of the invention can be used to inhibit growth of nosocomial bacteria, including bacteria that populate a typical hospital environment, or bacteria that are present on human skin, or bacteria that infect and form pus in wounds. Nosocomial infections are infections which become evident during a hospital stay or are related to a procedure performed in a hospital. These procedure-related infections often become evident after patients are discharged from the hospital. The most common nosocomial infections are urinary tract infections, surgical-site infections, pneumonia, and serious systemic infections, in which bacteria or fungi can be grown from blood.

Acquiring a microbe in a hospital does not cause a nosocomial infection itself. It is often stated that a patient 'contracted' a hospital bug and the surgical wound was infected. However, the issue is more complex.

The development of a nosocomial infection is a chain of events, which is influenced by the microbe, transmission route, and patient him/herself, i.e., his/her underlying illness, resistance to infections, and treatment of the underlying illness. Most nosocomial infections are caused by microbes which are otherwise present in the microbial flora on the skin or mucous membranes of the patient. To a lesser extent, microbes originate from outside the body: another patient, staff, or hospital environment. In addition, the microbial flora of the patient often change during the hospital stay, mostly due to anti-microbial treatment of particular components of the flora thereby often modifying the relationships of the various components. Modern treatments often necessitate the use of intravenous catheters, urinary catheters, respirators, hemodialysis, complicated operations, cortisone therapy and other factors, which depress resistance mechanisms and make patients susceptible to infections.

Institutional patients frequently develop nosocomial infections that are caused by normal flora colonizing the patient at the time of admission, or by exogenous pathogens that are acquired and subsequently colonize the patient after admission, e.g., to the hospital. A variety of strategies have been used either to prevent colonization from occurring, to eradicate colonizing organisms, or to prevent the progression from colonization to infection. These strategies include implementation of infection control measures designed to prevent acquisition of exogenous pathogens, eradication of exogenous pathogens from patients or personnel who have become colonized, suppression of normal flora, prevention of colonizing flora from entering sterile body sites during invasive procedures, microbial interference therapy, immunization of high-risk patients, and modification of antibiotic utilization practices. Strategies that require widespread use of antimicrobial agents to suppress or eradicate colonizing organisms tend to promote emergence of multidrug-resistant pathogens. Thus, a large number of potential infectious diseases lurk in environments where medical treatment is provided.

The methods and compositions of the invention are used to inhibit growth of gram negative or gram positive bacteria. Gram positive bacteria include, e.g., Staphylococcus (pyogenic), Enterococcus (opportunistic), Streptococcus, Enterococcus, Bacillus, Micrococcus, Mycobacterium, Corynebacterium, and Clostridium. Gram negative bacteria include e.g., Pseudomonas (pyogenic), E. coli (opportunistic), Salmonella (opportunistic), Campylobacter (opportunistic), Proteus (pyogenic), Klebsiella (opportunistic), Enterobacter (pyogenic), Citrobacter (pyogenic), Gram negative non-fermenter rods (e.g., Acinetobacter), and Shigella. The pyogenic cocci are spherical bacteria that cause various suppurative (pus-producing) infections in animals. Included are the Gram-positive cocci Staphylococcus aureus, Streptococcus pyogenes, and Streptococcus pneumoniae, and the Gram-negative cocci, Neisseria gonorrhoeae, and N. meningitidis. In terms of their phylogeny, physiology, and genetics, these genera of bacteria range from very near to very far in similarity. See, e.g., Garrity, et al. (eds. 2001) Bergy's Manual of Systematic Bacteriology Springer, NY.

The Gram-positive cocci are the leading pathogens of humans. It is estimated that they produce at least a third of all the bacterial infections of humans, including strep throat, pneumonia, food poisoning, various skin diseases, and severe types of septic shock. The Gram-negative cocci, notably the Neisseriae, cause gonorrhea and meningicoccal meningitis.

### C. Antibiotic resistant bacteria

The methods and compositions of the invention are used to inhibit growth, particularly of antibiotic resistant bacteria. For example, numerous bacterial pathogens of great importance to mankind have become multi-drug resistant (MDR), and these MDR strains have spread rapidly around the world. As a result, hundreds of thousands of people now die each year from infections that could have been successfully treated by antibiotics just 4-5 years earlier. See, e.g., Kunin (1993) Annals of Internal Medicine 118:557-561; and Neu (2002) Science 257:1064-73. In the case of MDR tuberculosis, e.g., immunocompromised as well as non-immunocompromised patients in our era are dying within the first month or so after the onset of symptoms, despite the use of as many as 11 different antibiotics.

Medical authorities have described multi-drug resistance not just for TB, but for a wide variety of other infections as well. Some infectious disease experts have termed this situation a "global crisis". In fact, efforts at developing new antibiotics are rather limited. A search is underway for alternative modes and novel mechanisms for treating these MDR bacterial infections.

Genetic variability in bacteria may also be created by acquisition of foreign DNA carried by plasmids, bacteriophages, or transposable genetic elements. An example of this phenomenon is the spread of a tetracycline-resistant transposon among Neisseria gonorrhoeae, Mycoplasma hominis, and Ureaplasma urealyticum. These mechanisms allow bacteria the potential to develop resistance to a conventional antibiotic. See Beers and Borkow (eds. 2003) The Merck Manual (17th ed.) Merck.

### D. Diagnosis of bacterial population, colonization, or infection

The diagnosis of bacterial colonization or infections assists in understanding the basis of infectious disease pathological symptoms. In particular, the detection and characterization of the local flora can be useful to determine the components and effects attributable to presence of infectious diseases. The genetic composition of the various strains and the interactions between strains and the host contribute to the resulting microbiological environment.

Initial diagnosis of potential or actual infectious agents, e.g., bacteria, typically leads to treatment strategies and methods. Thus, the ability to diagnose a bacterial infection can be used to identify the causative agent and treatment methods which can be appropriate to the specific infection. Methods of diagnosing bacterial infections are known to those of skill in the art, see, e.g., MacFaddin (2000) Biochemical Tests for Identification of Medical Bacteria (3d ed.) Lippincott, Williams & Wilkins, ISBN: 0683053183; Balows and Balows (1978) Biotyping in the Clinical Microbiology Laboratory Thomas Pubs.; Park, et al. (2003) J. Clin. Microbiol. 41:680-688; and Marks and Sharp (2000) J. Chem. Tech. and Biotech. 75:6-17.

The present invention also provides methods to diagnose bacterial populations or infections using anti-bacterial phages. The method is based, in large part, on specific interactions between an anti-bacterial phage and a target bacterium. Those of skill will recognize methods to label anti-bacterial phages and to use labeled anti-bacterial phages to detect a target bacterium in a biological sample from a subject suspected of having a bacterial infection. In particular, certain engineered constructs may be attached to specificity conferring tail components which provide high detectability, e.g., like antibody molecules attached to enzymes. Such tail-enzyme or tail-label constructs may take advantage of high turnover reactions to provide strong signals and high detection senstivity upon target bacterium interaction. And the specificity reagents may allow imaging strategies to localize the distributions of bacterial populations.

### IV. ANTI-BACTERIAL PHAGE

Anti-bacterial phages of the present invention are useful to treat bacterial infections caused by a target bacterium. In particular, because the anti-bacterial phages are unable to replicate in the target bacterium, the anti-bacterial phages can be administered in a defined dose.

Anti-bacterial phages are also particularly useful as anti-bacterial agents in an environment where bacterial growth is not desired or is considered to be harmful. For example, anti-bacterial phage preparations can be used to sterilize, including medical settings, operating room suites, food preparation areas, particularly areas where raw meat, e.g., beef, lamb, poultry, pork, or fish is handled. They may also be used to sterilize heat sensitive objects, medical devices, and tissue implants, including transplant organs.

### A. Methods to diminish replication activity of anti-bacterial phages in a target bacterium

Non-replicating phage constructs can be generated by making intact phage, and removing or inactivating the genetic material. Methods for removing the nucleic acid include, e.g., osmotic shock, freeze thaw, chemical treatment, or mechanical removal. Such may destroy the nucleic acid or allow it to escape. The phage coat may reassemble and reseal, or the DNA containing head segment of a phage may be lost. In many cases, the attaching and killing functions of the fragmented phage will be retained, while the genetic capacity of the composition is absent. Alternatively, the intact phage may be subjected to shear, and the separated tails purified away from other fragments.

Osmotic shock of phage may be performed, e.g., with salts or sugars. Freeze-thaw cycles of phage may result in mechanical or other fragmentation forces which allow for functional separation of the attaching/killing functions (e.g., provided by phage tails) and the genetic replication function. Chemical treatments of phage have also been observed to fragment the phage, e.g., treatment with LiCl or other salts; EDTA and/or other chelating agents; organic salts; amino acids; and reducing agents. Mechanical methods of fragmenting phage are available, including, e.g., shearing, lyophilization, sonication, microwave treatment, and others.

Other methods may be used for inactivating phage nucleic acid, e.g., UV irradiation, DNA fragmentation, chemical destruction (e.g., by D-glucosamine treatment), or ferrous ammonium sulfate. DNA modifying reagents can destroy the functional capacity of nucleic acids in phage, by preventing replication of the nucleic acid itself, by preventing assembly of an intact phage, by preventing release of phage from an infected bacterium, or by preventing the replication of genetically competent phage. Methods of chemical destruction of phage nucleic acids are found in the following references: Watanabe, et al. (1985) Agric. Biol. Chem. 49:63-70; Kashige, et al. (1994) Carbohydr. Res. 257:285- 291; Kakita, et al. (1995) Microbiol. Immunol. 39:571-576; Yamaguchi, et al. (1996) Biol. Pharm. Bull. 19:1261-1265; Kakita, et al. (1997) Biosci. Biotechnol. Biochem. 61:1947-1948; Yamaguchi, et al. (1998) Biol. Pharm. Bull. 21:205-209; Yamaguchi, et al. (1999) Tetrahedron 55:675-686; Watanabe, et al. (2000) Lett. Appl. Microbiol. 31:52-56; Kashige, et al. (2000) Biol. Pharm. Bull. 23:1281-1286; and Kashige, et al. (2001) Curr. Microbiol. 42:184-189.

In another embodiment, a replication incompetent phage lacks detectable nucleic acid component entirely. Such include partial phage, e.g., which lack the nucleic acids or the structural compartments which contain the nucleic acid component. These have been referred to as "ghosts" in certain studies on phage structure and the components functionally required to achieve infection processes. There are various methods for generating these constructs. Intact phage may be fragmented, and the tail portions which are involved in the binding and killing of target bacteria are often retained. The phage particles may be harvested from their infective cycles before the phage are completely assembled according to the genetic program for production and assembly of the phage within the bacterial host. The phage can be harvested after tail assembly, but temporally before attachment of the heads which contain genetic material.

Other replication incompetent phage can have disabled or incomplete phage genomes, e.g., prophages. Such phage may be intact, but lack critical parts of the genome, e.g., critical replication or assembly proteins. Simple embodiments include phage with genetic lesions or insertions in one or more critical genes. More complex embodiments include phage with termination codons in critical genes, which prevent expression or function of the gene products. Significant genetic deletions are also available, for which reversion mutations should be extremely rare. However, many of the genetically deficient phage may need to be produced with helper phage, or special complementing production host systems to provide the mutated function. These production host systems can be made by transforming them with genes encoding the deficiencies in the phage, e.g., to complement deficiencies in those hosts. Conversely, phage may be provided with additional functions which prevent replication, in which host production systems may inactivate those functions, e.g., restriction or phage exclusion systems. However, means to prevent genetic transfer between the host and the phage would be desired.

Moreover, yet another means to provide replication incompetent phage is to have phage with nucleic acids which are degraded before replication, e.g., susceptible to restriction enzymes encoded in the target bacteria, but which contain a gene which can kill the target bacterium before the phage DNA is destroyed. For example, one might package such a toxic protein / peptide into the head during packaging or in vitro (e.g., fusion with a DNA binding protein), which is then injected along with the DNA; or have a toxic protein expressed from a natural phage early promoter, which will be one of the earliest products, e.g., compatible holin or other membrane damaging proteins or peptides. Such can be generated by producing phage in modification incompetent host production cells with a suppressor of the killing gene, e.g., an antisense system. Or the phage may contain a "suicide" activity, e.g., a DNase gene, which causes destruction of both the phage and its host, but the phage is produced in a host production cell where the activity is ineffective, e.g., due to antisense message expression, or from lack of presence of a necessary cofactor.

Thus, specific production host strains may be important in production of the specific phage of the invention. For example, critical assembly genes may be deleted from the phage but provided by the production host and, in these embodiments, the phage are capable of being produced only in such host. Helper phage are another strategy to complement a deficiency (or addition), but means to prevent genetic recombination into the phage genome would be advantageous. Examples of phage mutations and complementing host or phage include, e.g., (1) phage comprising termination mutations and complementing host or helper phage comprising tRNA suppressors; (2) phage comprising mutations in genes critical for replication and complementing host or helper phage, e.g., comprising sequences that complement the mutation; (3) phage comprising insertion mutations and complementing host or helper phage, e.g., that comprise suppressors of the mutations; (4) phage comprising deletion mutations and complementing host or helper phage that, e.g., comprise suppressors of the mutations; and (5) phage comprising a suicide gene which kills both the phage and the target upon infection and insensitive or suppressor host producer or helper phage, e.g., containing antisense mRNA constructs.

Certain phage fragments can be assembled in vitro from purified protein components and used as anti-bacterial phage compounds, e.g., tail assemblies. Or the in vivo assembly of intact phage may be interrupted at a point where only tail assemblies have formed, e.g, before heads are attached. Alternatively, fragments may be made from purified proteins assembled in vitro, e.g., after large scale polypeptide synthesis methods. Particular scaffolding proteins or assembly activities may also need to be incorporated into the assembly vessels, though they may be needed only in very low stoichiometric quantities.

The tail structure has a tube, a sheath covering the tube, tail fibres, and base plate. Each of these structures are made of or contain different proteins. This structure in nature helps the phage to sense a bacterium, locate a receptor on its surface, bind to it, and then aid the release of DNA into the cell. The symmetry, stoichiometry, components, and composition can be modified to identify a minimum structure that is required for particular functions, e.g., specific adsorption (for diagnostic and imaging applications) or to kill a target cell. These likely do not need the entire natural structure.

For tailed phage, separation of nucleic acid-containing phage heads from phage tails can be performed to produce anti-bacterial phages, i.e., the isolated tails. Those of skill will recognize that phage head and tails can be separated using a variety of techniques, e.g., based on physical properties of the phage heads and tails, e.g., separation by size, charge, or other properties. For example, only phage heads contain nucleic acids and this can be exploited in separation by, e.g., gradient centrifugation. Other separation techniques, based largely on protein purification techniques follow.

### Solubility fractionation

Often as an initial step, an initial salt fractionation can separate many of the unwanted phage components (or proteins derived from the cell culture media) from the anti-bacterial phage tails The preferred salt is ammonium sulfate. Ammonium sulfate precipitates proteins and protein complexes by effectively reducing the amount of water in the protein mixture. Proteins and protein complexes then precipitate on the basis of their solubility. The more hydrophobic a protein or complex is, the more likely it is to precipitate at lower ammonium sulfate concentrations. A typical protocol includes adding saturated ammonium sulfate to a protein solution so that the resultant ammonium sulfate concentration is between 20-30%. This concentration will precipitate the most hydrophobic of proteins/complexes. The precipitate is then discarded (unless the protein of interest is hydrophobic) and ammonium sulfate is added to the supernatant to a concentration known to precipitate the protein of interest. The precipitate is then solubilized in buffer and the excess salt removed if necessary, e.g., through dialysis or diafiltration. Other methods that rely on solubility of proteins, such as cold ethanol precipitation, are well known to those of skill in the art and can be used to fractionate complex protein mixtures. See, e.g., *Methods in Enzymology.*

### Size differential filtration or sedimentation

The molecular weight of the anti-bacterial phage, e.g., the phage tail, can be used to isolate it from phage components of greater and lesser size using ultrafiltration through membranes of different pore size (for example, Amicon or Millipore membranes) or by sedimentation methods. As a first step using filtration, the phage mixture is ultrafiltered through a membrane with a pore size that has a lower molecular weight cut-off than the molecular weight of the protein/complex of interest. The retentate of the ultrafiltration is then ultrafiltered against a membrane with a molecular cut off greater than the molecular weight of the protein/complex of interest. The desired complex will pass through the membrane into the filtrate. The filtrate can then be chromatographed.

### Column chromatography

The phage components can also be separated from each other on the basis of its size, net surface charge, hydrophobicity, and affinity for ligands. In addition, antibodies raised against phage components can be conjugated to column matrices and the proteins immunopurified or immunoselected. These methods are well known in the art. It will be apparent to one of skill that chromatographic techniques can be performed at many different scales and using equipment from many different manufacturers (e.g., Pharmacia Biotech).

### C. Therapeutic treatment using anti-bacterial phages

The present invention can be applied across the spectrum of bacterial diseases, so that phage derived compositions are developed that are specific for each of the bacterial strains of interest. See, e.g., Merril, et al., Pat. App. US 2003/0026785 and Loomis and Fischetti, Pat. App. US 2002/0187136. In that way, a full array of compositions is developed for virtually all the bacterial (and other applicable) pathogens for man, his pets, livestock and zoo animals (whether mammal, avian, or pisciculture). Phage derived therapy will then be available, e.g, 1) as an adjunct to or as a replacement for those antibiotics and/or chemotherapeutic drugs that are no longer functioning in a bacteriostatic or bactericidal manner, e,g., due to the development of multi-drug resistance; 2) as a treatment for those patients who are allergic or intolerant to the antibiotics and/or chemotherapeutic drugs that would otherwise be indicated; and/or 3) as a treatment that has fewer or differently tolerable side effects than many of the antibiotics and/or chemotherapeutic drugs that would otherwise be indicated for a given infection.

Another embodiment of the present invention is the development of methods to treat bacterial infections in animals through phage derived therapy with the compositions described above. Hundreds of bacteriophages and the bacterial species they infect are known in the art. The present invention is not limited to a specific bacteriophage or a specific bacteria. Rather, the present invention can be utilized to develop bacteriophage derived compositions which can be used to treat many infections caused by their host bacteria.

While it is contemplated that the present invention can be used to treat most bacterial infections in an animal, it is particularly contemplated that the methods described herein will be very useful as a therapy (adjunctive or stand-alone) in infections caused by drug-resistant bacteria. Experts report (see, e.g., Gibbons (1993) Science 261:1036-38) drug-resistant bacterial species and strains which represent the greatest threats to mankind. See, e.g., Merril, et al., Pat. App. US 2003/0026785, pages 4-5; and Loomis and Fischetti, Pat. App. US 2002/10187136 page 5. These include, e.g., the clinically important members of the family Enterobacteriaceae, most notably, but not limited to the clinically important strains of Escherichia (most notably E. coli); Klebsiella (most notably K. pneumoniae); Shigella (most notably S. dysenteriae); Salmonella (including S. abortus-equi, S. typhi, S. typhimurium, S. newport, S. paratyphi-A, S. paratyphi-B, S. potsdam, and S. pollurum); Serratia (most notably S. marcescens); Yersinia (most notably Y. pestis); Comybacteria, and Enterobacter (most notably E. cloacae). Other important groups include Enterococci, most notably E. faecalis and E. faecium; Haemophilus, most notably H. influenzae; Mycobacteria, most notably M. tuberculosis, M. avium-intracellulare, M. bovis, and M. leprae; Neisseria gonorrhoeae and N. meningitidis; Pseudomonads, most notably P. aeuruginosa; Staphylococci, most notably S. aureus and S. epidermidis; Streptococci, most notably S. pneumoniae; and Vibrio cholera. In fact, these compositions will be particularly useful in treating macrophage intracellular bacterial infections such as tuberculosis, leprosy, Brucella, and Listeria. See, e.g., Broxmeyer, et al. (2002) J. Infect. Dis. 186:1155-60; and Greer (Oct. 22, 2002) TB and Outbreaks Week p.8*.,* both of which describe a system using Mycobacterium smegmatis, an avirulent mycobacterium, to deliver lytic phage into macrophages.

There are additional bacterial pathogens too numerous to mention that, while not currently in the state of antibiotic-resistance crisis, nevertheless make excellent candidates for treatment with these compositions, in accordance with the present invention. Thus, bacterial infections caused by bacteria for which there is a corresponding isolatable phage can often be treated using the present invention. See, e.g., Loomis and Fischetti, Pat. App. US 2002/0187136, page 5.

A phage strain capable of doing direct or indirect harm to a bacteria (or other pathogen) is contemplated as useful in the present invention. Thus, phages that are lytic, phages that are temperate but can later become lytic, and nonlytic phages that can deliver a product that will be harmful to the bacteria are all useful in the present invention. In many embodiments, lysogenic prophage may be excellent sources for screening for constructs having the desired specificity of killing, while not being capable of replicating.

Animals to be treated by the methods of the present invention include but are not limited to man, his domestic pets, livestock, work animals, pisciculture, and the animals in zoos and aquatic parks (such as whales and dolphins). Anti-bacterial phage can also be used to treat bacterial infections in plants, and potentially as diagnostic or sterilization reagents.

The compositions of the present invention can be used as a stand-alone therapy or as an adjunctive therapy, e.g., for the treatment of bacterial populations. Numerous antimicrobial agents (including antibiotics and chemotherapeutic agents) are known which would be useful in combination with these compositions for treating bacterial-based disorders. Examples of suitable antimicrobial agents and the bacterial infections which can be treated with the specified antimicrobial agents are known. See, e.g., Merril, et al., Pat. App. US 2003/0026785, page 5. However, the present invention is not limited to the antimicrobial agents listed, as one skilled in the art could easily determine other antimicrobial agents useful in combination with these compositions.

### D. Methods to identify anti-bacterial phages

Often, a method to identify an anti-bacterial phage will begin by identifying a target bacterium. Methods to identify a phage that infects a target bacterium, e.g., a wild-type, naturally occurring phage, are known to those of skill in the art. The methods described herein can be used to isolate, identify, or produce a form of the wild-type naturally occurring phage that kills the target bacterium, but lacks replication activity in the target bacterium, i.e., an anti-bacterial phage. Thus, the present invention allows for production of therapeutically useful compositions derived from the broad availability of natural phage. Moreover, the realization that phage parts may be sufficient to provide many of the functional properties of phage leads to the combinatorial mixing of components of phage which normally do not interact. By mixing components which normally do not interact, the potential to generate new specificities arises. This presents the possibility of using mutagenesis strategies to create tail-like specificities of extraordinarily broad or narrow specificities. And may compete with antibodies as a new technology to generate specificity reagents of high affinity, selectivity, and of highly predictable functions.

For example, the specificity of phage adsorption to cell surface receptors has been well studied in Escherichia coli and other Gram-negative bacteria. Major outer membrane components which determine the structure and the barrier function of the membrane of Gram-negative bacteria are receptors for many bacteriophages. LPS, the major component of the outer membrane of Enterobacteria, can be used by some phages with wide host range specificity. The other component of the outer membrane frequently used as a phage receptor component is the OmpA protein. Different sites of the OmpA protein are targetted by different phages, particularly of the T-even group. A large group of phage receptors are the porin proteins, which are discovered in 32 species of bacteria. In Gram-positive bacteria, phage adsorption almost always involves the cell surface carbohydrates and specific studies have been carried out for phages of Lactococcus species. Most staphylococcal phages contact the teichoic acids on the cell surface.

Because phage tails have peculiar symmetries, typically 4-fold or 6-fold, the binding characteristics of the intact tail are different from the individual protein components. Thus, the high local density of high affinity binding sites to the target receptor on the bacterial membrane should provide certain properties analogous to "tetramer" constucts using the T-cell receptor. However, with the 6-fold symmetry, the localized affinity may be extraordinarily high, even if the affinity of each interaction is low. And mixing of different binding domains may allow for interesting reagents which detect spatially constrained target features, or "epitopes".

A number of other interesting properties of the anti-bacterial phage of this invention may be different from the parental intact phage from which they may be derived. For instance, the anti-bacterial phage may infect both restriction-modification permissive and resistant target bacteria. The mechanism of killing may be different such that the restriction-modification activities of the target are irrelevant to the anti-bacterial phage. The intact phage typically kill only upon lysis of the target, which occurs after DNA replication and phage replication. In contrast, the anti-bacterial phage often kill essentially upon binding to the specific receptors, e.g., due to failure of resealing of the cell membrane after piercing to allow phage DNA entrance into the target cell. Thus, certain formulations or combinations with other antimicrobials are incompatible with the parental phage killing mechanism, which are not limited from the different kinetics and mechanism of killing by the anti-bacterial phage. Also, the anti-bacterial phage can often infect and kill target bacteria which possess superinfection-immunity to the parental phage, as shown in the Examples below.

In addition, physiology or genetic and protein engineering strategies may be applied to change the pharmacokinetics and pharmacodynamics of the tail constructs. PEG, liposomes, or colloidal carriers may affect the clearance mechanisms and processes. Changes to domains which regulate clearance, lifetime, body compartment accessibility, solubility properties, absorption, administration, stability, and other physical or physiological properties may be applied. Strategies for engineering protein stability include, e.g., recognition that tight packing of buried residues in a protein is an important determinant of protein stability (see Baldwin and Matthews (1994) Curr. Opin. Biotech. 5:396-402; Hubbard and Argos (1995) Curr. Opin. Biotech. 6:375-381; Richards and Lim (1993) Quart. Rev. Biophys. 26:423-498), as are compactness and efficient packing of hydrophobic residues (see Russell and Taylor (1995) Curr. Opin. Biotech. 6:370-374). Protein engineering to increase packing density through mutagenesis has been reported to lead to greater stability in the case of ribonuclease H1 (Ishikawa, et al. (1993) Biochemistry 22:6171-178), T4 lysozyme (Anderson, et al. (1993) Protein Sci. 2:1285-290, and ì repressor (Lim, et al. (1994) Proc. Nat'l Acad. Sci. USA 91:423-427; and Lim, et al. (1992) Biochemistry 5:4324-333).

### V. ADMINISTRATION

The route of administration and dosage will vary with the target bacteria, the site and extent of colonization (e.g., local or systemic), and the subject being treated. The routes of administration include but are not limited to: oral, aerosol or other device for delivery to the lungs, nasal spray, ocular, eye drops, intravenous (IV), intramuscular, intraperitoneal, intrathecal, vaginal, rectal, topical, lumbar puncture, intrathecal, and direct application to the brain and/or meninges. Excipients which can be used as a vehicle for the delivery of the phage will be apparent to those skilled in the art. For example, anti-bacterial phage could be in lyophilized form and be dissolved just prior to administration, e.g., by IV injection. The dosage of administration is contemplated to be in the range of about 1 thousand to about 10 trillion/kg/day, and preferably about 1 trillion/kg/day, and may be from about 106 killing units/kg/day to about 1013 killing units/kg/day, but may vary upon route of administration.

Methods to evaluate killing capacity are similar to methods used by those of skill to evaluate intact replicating phage, i.e., plaque forming units or pfu. Killing quantitation is more distinct, however, since the non-replicating phage will not normally form plaques on bacterial host lawns. Thus, serial dilution methods to evaluate the quantity of "killing" units are preferably used in place of standard pfu. The particular method used to establish killing units should not critical to the invention. Serial dilutions of bacterial cultures exposed to the killing compositions can quantitate killing units. Alternatively, comparing total bacterial counts with viable colony units can establish what fraction of bacteria are actually viable, and by implication, what fraction have been susceptible to the killing constructs.

The phage are typically administered in amounts or until successful elimination of the pathogenic bacteria is achieved. Thus the invention contemplates single dosage forms, as well as multiple dosage forms of the compositions of the invention, as well as methods for accomplishing delivery of such single and multi-dosage forms, including sustained release.

With respect to aerosol administration to the lungs, the phage composition is. typically incorporated into an aerosol formulation specifically designed for administration to the lungs by inhalation. Many such aerosols are known in the art, and the present invention is not limited to any particular formulation. An example of such an aerosol is the Proventil□ inhaler manufactured by Schering-Plough, the propellant of which contains trichloromonofluoromethane, dichlorodifluoromethane, and oleic acid. The concentrations of the propellant ingredients and emulsifiers are adjusted if necessary based on the phage composition being used in the treatment. The number of phage to be administered per aerosol treatment will be typically in the range of 106 to 1013 killing units, and preferably 1012 killing units. **[KLB: this doesn't match the dose/kg indicated above]**

Methods to evaluate killing capacity are similar to many methods used in working with intact replicating phage. In particular, killing quantitation is more difficult since the non-replicating phage will not form plaques on bacteria, though mixed lawns may be useful. Thus, serial dilution methods to evaluate the quantity of "killing" units will be performed similarly to standard pfu (plaque forming units), but cannot make use of the killing and amplification which occurs on a bacterial host lawn. Serial dilutions of bacterial cultures exposed to the killing compositions can quantitate killing units. Alternatively, comparing total bacterial counts with viable colony units can establish what fraction of bacteria are actually viable, and by implication, what fraction have been susceptible to the killing constructs.

Methods to evaluate the replication capacity of a construct can use normal plaque forming assays. Typically, the inactivation will decrease the replication capacity by at least 3 fold, and may affect it by 10, 30, 100, 300, etc., to many orders of magnitude. Preferred genetic inactivation efficiencies may be 2, 3, 4, 5, 6, 7, 8, or more log units.

### VI. FORMULATIONS

The invention further contemplates pharmaceutical compositions comprising at least one bacteriophage of the invention provided in a pharmaceutically acceptable excipient. The formulations and pharmaceutical compositions of the invention thus contemplate formulations comprising an isolated bacteriophage specific for a bacterial host; a mixture of two, three, five, ten, or twenty or more bacteriophage that infect target bacterial hosts; and a mixture of two, three, five, ten, or twenty or more bacteriophage that infect different bacterial hosts or different strains of the same bacterial host. (e.g., a mixture of bacteriophage that collectively infect and inhibit the growth of multiple strains of Staphylococcus aureus). In this manner, the compositions of the invention can be tailored to the needs of the patient, as an individual or as a member of a defined set of patients.

By "therapeutically effective dose" herein is meant a dose that produces effects for which it is administered. The exact dose will depend on the purpose of the treatment, and will be ascertainable by one skilled in the art using known techniques (e.g., Ansel, et al. (1992) Pharmaceutical Dosage Forms and Drug Delivery Lieberman, Pharmaceutical Dosage Forms (vols. 1-3), Dekker, ISBN 0824770846, 082476918X, 0824712692, 0824716981; Lloyd (1999) The Art, Science and Technology of Pharmaceutical Compounding*;* and Pickar (1999) Dosage Calculations). Adjustments, e.g., for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

Various pharmaceutically acceptable excipients are well known in the art. As used herein, "pharmaceutically acceptable excipient" includes a material which, when combined with an active ingredient of a composition, allows the ingredient to retain biological activity and without causing undue disruptive reactions with the subject.

Exemplary pharmaceutically carriers include sterile aqueous or non-aqueous solutions, suspensions, and emulsions. Examples include, but are not limited to, standard pharmaceutical excipients such as a phosphate buffered saline solution, water, emulsions such as oil/water emulsion, and various types of wetting agents. Examples of non-aqueous solvents are propylene glycol, polyethylene glycol, vegetable oils such as olive oil, and injectable organic esters such as ethyl oleate. Aqueous carriers include water, alcoholic/ aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like.

A composition comprising a bacteriophage of the invention may also be lyophilized using means well known in the art, for subsequent reconstitution and use according to the invention.

Also provided are formulations for liposomal delivery, and formulations comprising microencapsulated bacteriophage, e.g., which may provide sustained release kinetics or allow oral ingestion to pass through the stomach. Compositions comprising such excipients are formulated by well known conventional methods (see, e.g., Remington's Pharmaceutical Sciences, Chapter 43, 14th Ed., Mack Publishing Col, Easton PA 18042, USA).

In general, the pharmaceutical compositions can be prepared in various forms, such as granules, tablets, pills, suppositories, capsules (e.g. adapted for oral delivery), microbeads, microspheres, liposomes, suspensions, salves, lotions, and the like. Pharmaceutical grade organic or inorganic carriers and/or diluents suitable for oral and topical use can be used to make up compositions comprising the therapeutically-active compounds. Common diluents include aqueous media, vegetable and animal oils, and fats. Stabilizing agents, wetting and emulsifying agents, salts for varying the osmotic pressure, or buffers for securing an adequate pH value may be included.

The pharmaceutical composition can comprise other components in addition to the phage. The pharmaceutical compositions may comprise a plurality of phage, e.g., two, three, five, or ten or more different phage, where the different phage may be specific for the same or different target bacteria. The pharmaceutical composition can contain multiple (e.g., at least two or more) defined phage, wherein at least two of the phage in the composition have different bacterial target specificity. In this manner, the phage composition can be adapted for treating a mixed infection of different bacteria, e.g., by selecting different groups of phage of differing specificity so as to contain at least one phage for each bacteria (e.g., strain, species, etc.) suspected or likely to be present in the affected site. As noted above, the phage can be administered in conjunction with other therapeutic agents, such as an inflammatory or conventional antimicrobial agent. In some embodiments, it may be desirable to administer the phage and another therapeutic, e.g., antibiotic, within the same formulation.
**[KLB: where should we describe the issue of broad specificity of antibiotics and potential narrow specificity of our compositions, which will provide various advantages???]**

### VII. METHODOLOGY

Some aspects of practicing the present invention involve well-known methods, e.g., general clinical microbiology, general methods for handling bacteriophage, and general fundamentals of biotechnology, principles and methods. References for such methods are listed below and are herein incorporated by reference for all purposes.

### A. General clinical microbiology

General microbiology is the study of the microorganisms. See, e.g., Sonenshein, et al. (eds. 2002) Bacillus Subtilis and Its Closest Relatives: From Genes to Cells Amer. Soc. Microbiol., ISBN: 1555812058; Alexander and Strete (2001) Microbiology: A Photographic Atlas for the Laboratory Benjamin/Cummings, ISBN: 0805327320; Cann (2001) Principles of Molecular Virology (Book with CD-ROM; 3d ed.), ISBN: 0121585336; Garrity (ed. 2001) Bergey's Manual of Systematic Bacteriology Volume 1: The Archaea, Cyanobacteria, Phototrophs & Deeply (2d ed.) Springer Verlag, ISBN: 0387987711; Salyers and Whitt (2001) Bacterial Pathogenesis: A Molecular Approach (2d ed.) Amer. Soc. Microbiol., ISBN: 155581171X; Tierno (2001) The Secret Life of Germs: Observations and Lessons from a Microbe Hunter Pocket Star, ISBN: 0743421876; Block (ed. 2000) Disinfection, Sterilization, and Preservation (5th ed.) Lippincott Williams & Wilkins Publ., ISBN: 0683307401; Cullimore (2000) Practical Atlas for Bacterial Identification Lewis Pub., ISBN: 1566703921; Madigan, et al. (2000) Brock Biology of Microorganisms (9th ed.) Prentice Hall, ASIN: 0130819220; Maier, et al. (eds. 2000) Environmental Microbiology Academic Pr., ISBN: 0124975704; Tortora, et al. (2000) Microbiology: An Introduction including (TM) Website, Student Tutorial CD-ROM, and Bacteria ID CD-ROM (7th ed.) Benjamin/Cummings, ISBN 0805375546; Demain, et al. (eds. 1999) Manual of Industrial Microbiology and Biotechnology (2d ed.) Amer. Soc. Microbiol., ISBN: 1555811280; Flint, et al. (eds. 1999) Principles of Virology: Molecular Biology, Pathogenesis, and Control Amer. Soc. Microbiol., ISBN: 1555811272; Murray, et al. (ed. 1999) Manual of Clinical Microbiology (7th ed.) Amer. Soc. Microbiol., ISBN: 1555811264; Burlage, et al. (eds. 1998) Techniques in Microbial Ecology Oxford Univ. Pr., ISBN: 0195092236; Forbes, et al. (1998) Bailey & Scott's Diagnostic Microbiology (10th ed.) Mosby, ASIN: 0815125356; Schaechter, et al. (eds. 1998) Mechanisms of Microbial Disease (3d ed.) Lippincott, Williams & Wilkins, ISBN: 0683076051; Tomes (1998) The Gospel of Germs: Men, Women, and the Microbe in American Life Harvard Univ. Pr., ISBN: 0674357078; Snyder and Champness (1997) Molecular Genetics of Bacteria Amer. Soc. Microbiol., ISBN: 1555811027; Karlen (1996) Man and Microbes: Disease and Plagues in History and Modern Times Touchstone Books, ISBN: 0684822709; and Bergey (ed. 1994) Bergey's Manual of Determinative Bacteriology (9th ed.) Lippincott, Williams & Wilkins, ISBN: 0683006037.

### B. General methods for handling bacteriophage

General methods for handling bacteriophage are well known, see, e.g., Snustad and Dean (2002) Genetics Experiments with Bacterial Viruses Freeman; O'Brien and Aitken (eds. 2002) Antibody Phage Display: Methods and Protocols Humana; Ring and Blair (eds. 2000) Genetically Engineered Viruses BIOS Sci. Pub.; Adolf (ed. 1995) Methods in Molecular Genetics: Viral Gene Techniques vol. 6, Elsevier; Adolf (ed. 1995) Methods in Molecular Genetics: Viral Gene Techniques vol. 7, Elsevier; and Hoban and Rott (eds. 1988) Molec. Biol. of Bacterial Virus Systems (Current Topics in Microbiology and Immunology No. 136) Springer-Verlag.

### C. General fundamentals of biotechnology, principles and methods

General fundamentals of biotechnology, principles and methods are described, e.g, in Alberts, et al. (2002) Molecular Biology of the Cell (4th ed.) Garland ISBN: 0815332181; Lodish, et al. (1999) Molecular Cell Biology (4th ed.) Freeman, ISBN: 071673706X; Janeway, et al. (eds. 2001) Immunobiology (5th ed.) Garland, ISBN: 081533642X; Flint, et al. (eds. 1999) Principles of Virology: Molecular Biology, Pathogenesis, and Control Am. Soc. Microbiol., ISBN: 1555811272; Nelson, et al. (2000) Lehninger Principles of Biochemistry (3d ed.) Worth, ISBN: 1572599316; Freshney (2000) Culture of Animal Cells: A Manual of Basic Technique (4th ed.) Wiley-Liss; ISBN: 0471348899; Arias and Stewart (2002) Molecular Principles of Animal Development Oxford University Press, ISBN: 0198792840; Griffiths, et al. (2000) An Introduction to Genetic Analysis (7th ed.) Freeman, ISBN: 071673771X; Kierszenbaum (2001) Histology and Cell Biology, Mosby, ISBN: 0323016391; Weaver (2001) Molecular Biology (2d ed.) McGraw-Hill, ISBN: 0072345179; Barker (1998) At the Bench: A Laboratory Navigator CSH Laboratory, ISBN: 0879695234; Branden and Tooze (1999) Introduction to Protein Structure (2d ed.) Garland Publishing; ISBN: 0815323050; Sambrook and Russell (2001) Molecular Cloning: A Laboratory Manual (3 vol., 3d ed.), CSH Lab. Press, ISBN: 0879695773; and Scopes (1994) Protein Purification: Principles and Practice (3d ed.) Springer Verlag, ISBN: 0387940723.

It must be noted that as used herein and in the appended claims, the singular forms "a", "and", and "the" include plural referents unless the context clearly dictates otherwise. Thus, e.g., reference to "a bacteriophage" includes a plurality of such bacteriophage and reference to "the host production bacterium" includes reference to one or more host production bacteria and equivalents thereof known to those skilled in the art, and so forth.

### EXAMPLES

### Example 1: Methods of inactivating or removing nucleic acid to make anti-bacterial phage.

### a. Osmotic shock treatment

Nucleic acids can be released from phage upon osmotic shock treatment. Phage are prepared and subjected to osmotic shock at an appropriate temperature, e.g., low temperature, and for an appropriate amount of time, e.g., 1-60 minutes, depending upon the phage type and strain. See, e.g., Minagawa (1977) Virology 76:234-245 (NaCl shock) or Szewczyk and Skorko (1981) Biochim. Biophys. Acta 662:131-137 (sucrose shock). Other osmotic agents can be used, and the shock medium may be supplemented with, e.g., appropriate amounts of nucleases, proteases, protease inhibitors, etc.

After the removal of the nucleic acid, the intact, replication competent phage are removed from the preparation. Such can be achieved, e.g., by size or weight based separation methods. A preferred method is density separation, as the phage particles lacking nucleic acid differentially separate from intact particles. The inactivated anti-bacterial phage are collected and confirmed still specific and capable of killing, and the intact phage my be collected or discarded. Intact phage may be useful as starting materials for a second shock treatment, or for diagnostic or other uses where the replication capacity may be useful.

### b. EDTA treatment

Phage subjected to EDTA treatment yield DNA deficient phage. EDTA treated phage retain target bacterium binding capacity, and kill target bacteria. See, e.g., Konopa and Taylor (1975) Biochim. Biophys. Acta 399:460-467. The treated phage are subjected to purification methods to separate intact, replication competent phage from anti-bacterial phage that lack nucleic acid using e.g., density separation.

### c. Amino acid treatment

Exposure to various amino acids have been reported to affect phage replication competence. See Murata, et al. (1974) Agr. Biol. Chem. 38:477-478. Various amino acids may be used, and the time and conditions to which the phage are exposed are optimized for the specific phage and target host pair. The treated phage are subjected to purification methods to separate intact, replication competent phage from anti-bacterial phage that lack nucleic acid using e.g., density separation.

### d. Lyopholization treatment

Phage subjected to lyophilization become replication deficient, while retaining target bacterium killing capacity. See, e.g., Shapira and Kohn (1974) Cryobiology 11:452-464; and Clark and Geary (1973) Cryobiology 10:351-360. The treated phage are subjected to purification methods to separate intact, replication competent phage from anti-bacterial phage that lack nucleic acid using e.g., density separation.

### e. Microwave treatment

Exposure of bacteriophage to microwave irradiation can diminish the replication capacity of a phage. See, e.g., Kikita, et al. (1995) Microbiol. Immunol. 39:571-576; and Watanabe, et al. (2000) Lett. Appl. Microbiol. 31:52-56. The treated phage are subjected to purification methods to separate intact, replication competent phage from anti-bacterial phage that lack nucleic acid using, e.g., density separation.

### f. UV or X-ray irradiation treatment

Irradiation by UV or X-rays inactivates the replication capacity of phage. The treated phage are subjected to purification methods to separate intact, replication competent phage from anti-bacterial phage that lack nucleic acid using e.g., density separation.

### g. D-glucosamine treatment

D-glucosamine treatment of phage inactivates the replication competence of phage, while retaining the killing capacity. See, e.g., Watanabe, et al. (1985) Agr. Biol. Chem. 49:63-70; and Yamaguchi, et al. (1998) Biol. Pharm. Bull. 21:205-209. The reagent introduces breaks into the nucleic acid, thereby preventing its replication. The treated phage are subjected to purification methods to separate intact, replication competent phage from anti-bacterial phage that lack nucleic acid, e.g., using density separation.

### h. Isolation of tails

Phage tails are most directly obtained from induction of a prophage-containing bacterial strain using mitomycin C or similar DNA-damaging chemicals. The prophage is induced and undergoes replication, while tails are assembled. No heads are synthesized because of a defect in a head assembly pathway. Thus, upon lysis, many tails are produced but no heads.

A similar "tails" production strategy involves isolating phage with conditional mutations in head gene expression or function. The mutant phage are grown up until the last step in production on suppressor hosts or under suppressing conditions. The simplest idea is to isolate temperature-sensitive mutants of the phage which have a temperature sensitive-mutation in an essential head gene. These mutant phages can be then grown under non-suppressing conditions, to generate the tail preparations.

### i. Specific phage and host combinations

Phage which have defects in genes necessary for packaging genetic material will produce phage assemblies which lack genetic material, e.g., prophages. Variants can be isolated which exhibit mutations, e.g., point, deletion, insertion, etc., in critical head structure or head assembly genes, but, which do not affect production of the tail portions that are responsible for the binding and killing functions.

Lytic phage that are conditional producers of the nucleic acid packaging components can be isolated or engineered. For example, termination codons or temperature sensitive mutants can be identified or engineered to produce the phage particles under permissive conditions or in permissive hosts. Termination suppressor hosts or temperature sensitive hosts can allow production, while neither phage would be capable of replicating in the target bacterial hosts. Means to prevent transfer of the permissive factors into the phage genome are devised to minimize the possibility of recombination creating replication competent phage.

Alternatively, normal phage are produced in production hosts which are engineered to produce or assemble only the killing, e.g., tail, components. This is achieved, e.g., by blocking structural or assembly genes critical for assembly of the head components, e.g., by plasmids that express an antisense version of the target genes.

### j. Combination of methods

The methods a-i can be combined to produce and enrich for replication incompetent phage, i.e., anti-bacterial phage. In some embodiments, separation steps may be used to remove intact, replication competent phage. Further additional steps of nucleic acid removal or inactivation can be included to reduce the amounts of intact phage which may copurify with the replication incompetent anti-bacterial phage.

### Example 2: Phage tails from a lytic phage

P9042 and P954, exemplary bacteriophages isolated from a natural water source, were used to establish operability of the present strategy, These phage propagate in the strain Staphylococcus aureus and have been so designated based on a labeling system adopted to categorize and number phages in the Gangagen phage library. P9042 is a lytic phage, while P954 is a lysogenic phage. P9042 and P954 are examples of phages that were isolated from nature, and similar selection and isolation procedures should provide other phage with similar desired combinations of properties, as appropriate.

### A. Tail-specific activity assay for lytic phage tail preparations

S. simulans (10E7) cells were suspended in a volume of 100 µl. The suspension was treated with either the P9042 wild phage or the P9042 tail preparation, in a total volume of 200 µl. Each assay was performed in triplicate in a microtiter plate. The samples were incubated at 37° C for 20 min, the OD 630 absorbance checked for each sample, and the sample plated on an LB plate. The plates were incubated at 37° C overnight, and the residual c.f.u. (i.e., resistant cells) were quantitated.

### B. Prevention of translation of capsid message via antisense RNA

P9042 capsid gene expression was targeted by this method using a VegII promoter which allowed P9042 capsid antisense RNA to be produced constitutively and which thereby would inhibit translation of the P9042 capsid gene.

The P9042 capsid gene was cloned into a Staphylococcus aureus vector (designated pGMB300) in reverse orientation to the VegII promoter. This clone construct was designated pGMB331. This vector construct was engineered to provide the following features: an E. coli - S. aureus shuttle vector constructed by cloning S.aureus plasmid pC194 into the HindIII site of E.coli plasmid pRSetA; a constitutive vegetative promoter, VegII, which functions in both E. coli and S. aureus; a beta lactamase marker for selection in E. coli; and a chloramphenicol acyl-transferase marker for selection in S.aureus. See, e.g., Jankovic, et al, (2001) J. Bacteriology 183:580-586; and Horinouchi and Weisblum (1982) J. Bacteriology 150:815-825.

The host RN4220, a derivative of 8325-4, a prophage cured, restriction deficient S. aureus strain (see Kreiswirth, et.al (1983) Nature 305:709-712) was obtained from Dr. Richard Novick (Skirball Institute, New York).

The pGMB331 plasmid was introduced into electrocompetent-RN4220 using standard procedures. See Bio-Rad Micropulser instruction manual. Chloramphenicol resistant transformants were isolated, and the presence of the intact pGMB331 plasmid in the transformants was confirmed by P9042 capsid gene PCR. Selected transformants were grown at 37° C to OD600 of about 0.6. Cells were infected with P9042 phage at an input ratio of 0.5. After 40 min (latent period of P9042 is 40-45 minutes), the infected culture was centrifuged at 8000 rpm for 10 min. The supernatant was discarded and the cell pellet was washed with culture broth, then resuspended in 1/50 volume of culture broth. The cells were sonicated and the lysate centrifuged, and the supernatant was analyzed for the presence of phage tails.

Staphylococcus simulans was used as target host to determine the killing activity of P9042 tails. Phage P9042 is capable of infecting S. simulans but is incapable of propagating in this same host. These cells therefore exhibit receptors for attachment of the phage but do not allow replication of the phage (due to a resident restriction-modification system that inactivates the nucleic acids). A tail preparation is devoid of capsid and associated nucleic acids and is thus not subject to this phenomenon of DNA restriction or degradation. However, as the tail possesses essentially the same receptor and machinery for effective adsorption and infection to target bacteria, the action of a capsid-less phage "tail" on the bacterial cells renders the tail assemblies "non-viable", or replication deficient.

The antibacterial phage product (tails) can kill both restriction-modification permissive and resistant target bacteria, whereas the parent bacteriophage nucleic acid is inactivated by restriction and is replication ineffective (both DNA replication and phage replication) in such bacteria.

The sonicated cell supernatant was also checked by routine plaque test to evaluate the presence of whole assembled P9042 phage that escaped the antisense-mediated inhibition. This analysis indicated that the antisense lysate exhibited about 2% of the residual colony forming units (c.f.u.) of the wild type phage lysate, indicating that the antisense strategy decreased the production of replication competent phage by 98%, or some two orders of magnitude. Electron microscope analysis of the samples confirmed the presence of tail-like structures.

P9042 phage in the wild phage lysate did not kill S. simulans at an input ratio as high as 10, whereas the antisense lysate decreased the c.f.u. on S. simulans by 98%. Thus, the soluble phage constructs maintained selectivity of killing, while exhibiting decreased replication capacity of the phage on that host.

### C. Harvesting of phage tails at a specific time point during the phage cycle

Upon infection, these phage follow the infective cycle comprising DNA synthesis; synthesis of tail proteins and capsid proteins; assembly of the respective tail proteins into tail assemblies and capsid proteins into head assemblies; and finally attachment of the tail assemblies with head assemblies to generate intact phage particles. Tail assemblies may be harvested by lysing host cells at a time point prior to the attachment of tail assemblies with head assemblies into intact phage particles.

The host bacteria RN4220 was grown at 37° C to OD600 of 1.0. The host were infected with P9042 at a 0.5 multiplicity of infection (m.o.i.) at 37° C. Infected samples of host cells were removed after 15, 30, and 40 min and chilled. Each sample was centrifuged in cold at 8000 rpm for 10 min and the cell pellets were washed with LB. Each pellet was resuspended in 1/20th of initial volume of LB, and the samples sonicated. The supernatant was checked for the presence of tails as described. Electron microscope analyses of the samples confirmed the presence of tail-like structures.

From the 15 minute sample, the supernatant contained 69% of the tail killing activity of the full cycle assembled wild type phage. From the 30 minute sample, only 4% of the tail killing activity was found in the soluble fraction, and only 2% from the 40 minute sample. Thus, interrupting the normal assembly process before phage assembly is normally completed allows for isolation of a significant fraction of the tail proteins being assembled into killing tail assemblies. This also establishes that these tail assemblies can be purified away from head assemblies based on the temporal separation of tail assembly and phage assembly. This suggests that mutational variants may be selected, e.g., which extend the temporal separation of tail assembly from phage particle assembly, to improve both the yield and separation of tail assemblies from intact phage.

### EXAMPLE 3: Phage tails from a lysogenic phage

### A. Lysogenization of target phage P954

The non-pathogenic and prophage-free Staphylococcus aureus strain RN4220 was lysogenized with Phage P954 by infection at low input ratio and screening for colonies immune to P954 phage. Colonies resistant to P954 phage would carry at least one copy of the phage in the genome as a prophage, which confers the resistance to phage infection. The mechanism by which the presence of the prophage affords immunity to infection by the same and related phages is described in the literature.

An overnight culture of RN4220 was subcultured into 50 ml LB and grown to 0.5 OD600 at 37° C. The culture was then infected with P954 at a m.o.i. of 1, and incubated at 37° C for 20 min. The infected sample was centrifuged at 7000 rpm for 10 min. The supernatant was discarded and the pellet washed with 50 ml LB by resuspension and centrifugation at 7000 rpm for 10 min. The supernatant was discarded and the pellet resuspended in 50 ml LB. The resuspended cell pellet was diluted to plate 1000 c.f.u. onto LB plates. The resulting colonies were inoculated into 96 well plates and incubated overnight at 37° C. The overnight cultures were sub-cultured into 96 well plates and incubated at 37° C for two hr. Each of the 96 cultures were grown to a lawn, and 2 µl of P954 lysate was spread on each lawn and incubated at 30° C. After 3 h, plates were evaluated for lysis. Cells that were not susceptible to phage P954 were characterized as lysogenised (e.g., immune).

To select a lysogen carrying a single copy of prophage, several colonies were induced with mitomycin C for prophage release and screened for those colonies that yield low titers of phage, in the range of 10E6 to 10E7 per ml.

### B: Prophage induction and kinetics of phage production during lytic cycle following MitC induction

Phage titers of a lysogen, P954-RN4220#A, were monitored at different time points after Mitomycin C (MitC) induction. Tail preparation were made.

The lysogen was subcultured overnight, and grown to OD600 of 1.0 at 37° C. The culture was induced with 1 mg/ml of MitC at 37° C. Samples were removed at various time intervals, e.g., at 30 min, 60 min, 2 h, 3 h, and 4 h. Each sample was centrifuged at 7000 rpm for 10 min, and cell pellets resuspended in 1 ml of LB broth. The cell pellets were then lysed with glass beads. Phage titers were checked on a lawn of RN4220 target bacteria.

Intracellular phage were evaluated (pfu/ml) at each time point; at 0.5 h 1x10E6; at 1 h 5x10E6; at 2 h 1x10E7; at 3 h 2.5x10E7; and at 4 h 4x10E6. The culture was lysed after 4 h of MitC induction. Thus, no significant difference in phage titer was seen at different time points.

### C: Evaluation of tail based killing from lysogenized cell lysate supernatants

S. aureus strain B935 was used to evaluate the extent of tail based killing from lysates of lysogenized hosts. Lysates at various time points after MitC induction were analysed for tail based killing, as described.

The 2 hour sample contains phage tails that kill Staphylococcus aureus B935, while wild P954 phage is unable to kill the same strain even at input ratio of 100. Thus, the protective mechanism provided by the lysogenic integration exhibits selective immunity.

### D: Evaluation of tail preparations on a panel of lysogenic S. aureus isolates

Phage P954 is capable of infecting a spectrum of Staphylococcus aureus isolates, e.g., B935, B904, B913, B920, B903, B975, and B972 (the "P954-tail-killing test panel"); but is incapable of propagating in these isolates. Each of these isolates thus harbors receptors for attachment of the phage, but do not allow replication of the phage, e.g., due to a resident prophage. This immunity phenomenon is known as "immunity to superinfection" and is described extensively in literature. *See e.g.,* Bertani (1953) Genetics 38:653; Bertani and Bertani (1971) Adv. Genet. 16:199-237; Cam, et al. (1991) J. Bacteriol. 173:734-740; Csiszovszki, et al. (2003) J. Bacteriol. 185:4382-4392; Dhaese, et al. (1985) Mol. Gen. Genet. 200:490-492; Heinrich, et al. (1995) FEMS Microbiol. Rev. 17:121-126. Immunity to an infective phage is typically brought about by presence of a similar prophage that induces inactivating the incoming phage DNA. The presence of such a prophage in each of the above isolates was confirmed by PCR detection of P954 phage-specific DNA sequences in its genome.

A tail preparation is devoid of capsid protein and/or associated nucleic acids, and is therefore not subject to this phenomenon of inactivating incoming phage DNA. However, as the tail possesses substantially the same receptor and functional machinery for effective adsorption and infection, the action of a capsid-less phage "tail" assembly on the bacterial cells renders them incapable of either phage or DNA replication, e.g., providing the characteristic of "defined dose".

For testing P954 phage tail-based killing, some or all strains from the above panel were tested. The antibacterial phage product (tails) can kill many, and perhaps virtually all, target bacteria isolates that possess "superinfection-immunity" to the parent bacteriophage. In contrast, the parent bacteriophage is inactivated in such bacteria. Thus, the tail assembly may exhibit a broader target killing range than the natural parental phage.

P954-tail-killing test panel isolates of S. aureus (10E7) cells were suspended in a volume of 100 µl and treated with test samples, either P954 wild phage at an input ratio of 10 or P954 from 2 h lysate (tail preparation). Each assay was done in triplicate in a microtiter plate in a total assay volume of 200 µl. The cultures were incubated for 1 h at 37° C on a shaker at 200 rpm. OD630 absorbance of each sample was checked. Each sample was plated on an LB plate and incubated at 37° C overnight. Residual c.f.u., i.e., bacteria which avoided being killed, were determined.

Results are listed as follows: isolate (P954 intact phage residual % c.f.u., 2 h lysate tail preparation residual % c.f.u.). B935 (90, 3); B904 (95, 7); B913 (95, 11); B920 (98, 3); B903 (72, 0.3); B975 (90, 3); B972 (96, 1.3). Thus, in each case, the susceptibility of each isolate to the intact phage P954 is relatively low (less than 30%) than the susceptibility of each isolate to the lysate tail preparation (more than about 90%).

### EXAMPLE 4: Tail prepartions from a lysogenic phage P954

### A. Size of killing activity

To confirm that the killing activity was effected by tail sized particles and not low molecular weight proteins, e.g., P954 lysin, the tail prep made according to the protocol described above was subjected to a centrifugation spin using a 300 kDa cutoff membrane. Both the retentate and the filtrate were checked for target bacteria killing by the method described above. Reduction in the absorbance at 630 nm was taken as the measurement of target bacteria killing activity

P954 tail preparation was subjected to 300 kDa cutoff membrane and the retentate used for killing assay, and done in triplicate. The cell control and cells with phage exhibited essentially no killing (because the lysogenic phage imparts immunity). The retentate of 300 Kda exhibited about 60% decrease in A630, while the filtrate, e.g., particles of less than about 300 Kda size, exhibited essentially no decrease in A630.

These results indicate that only the 300 kDa retentate had target killing activity while no target killing activity was observed in the filtrate of the tail preparation. This indicates low molecular weight proteins/compounds (< 300 kDa) are not sufficient to effect the observed bacterial killing activity. It also indicates that the lysogenic phage impart immunity to infection with intact phage, but not to the tail preparations. Thus, the killing capacity of tail preparations is different from intact phage, which suggests a different mechanism of cell killing from intact phage.

The P954 lysin in the genomic DNA sequence should encode a protein of about 28 kDa. Thus, it should not be retained in the 300 Kda membrane. Since the retentate lacks killing activity, P954 phage lysin would not be sufficient to effect the killing activity observed. This is consistent with the killing being effected by large molecular weight tail structures.

### B. Time course of killing activity of tail preparations

To determine the time course of killing by the P954 tail preparation, susceptible cells were treated with the 300 kDa cutoff P954 tail preparation. Samples were removed at various time points and residual cell viability determined by plating.

| Results: | | CFUs | |
|---|---|---|---|
| Samples | B935 cell control | B935+ tail prep | B935+P954 phage |
| 5 minutes | 220 | 170 | ND |
| 10 minutes | 262 | 85 | ND |
| 20 minutes | 237 | 40 | ND |
| 25 minutes | 500 | 10 | ND |
| 35 minutes | 500 | 45 | 470 |

These results show that tail killing activity starts as early as 10 minutes under the assay conditions described, where the doubling time of the target bacteria is about 25 min.

### C. Characteristics of Gram positive tail preparations:

High molecular weight bacteriocins (like pyocins) are known to be susceptible to heat and resistant to trypsin. See Jabrane, et al. (2002) Appl. and Environ. Microbiol. 68:5704-5710; and Shimizu, et al. (1982) J. Virology 44:692-695. The effect of heat on intact P954 phage was evaluated. 10E8 pfu were heated at 65° C for 15 min, after which residual pfu were determined. The results showed that >99% inactivation of P954 phages resulted under the conditions described above, actually decreasing the phage titer by about 3-4 orders of magnitude.

Then, P954 tail preparations were subjected to the heat treatment, as mentioned above, and surviving target killing activity assayed. Results indicate that P954 tail preparations were sensitive to heat treatment.

### D. Effect of trypsin on P954 phage tail preparations

Trypsin from porcine pancreas (Sigma, USA; 2.5 mg/ml) was used. Suitable amounts of P954 phage tail preparations (10E8 phage) were treated with trypsin for 1 h at 37° C. Surviving phage titers were determined on target propagating host.

A P954 tail preparation was subjected to 300 kDa cutoff membrane and the retentate used for the killing assay, as described above. The A630 for the cells alone was about .50; for cells with P954 phage about .43; for cells with P954 tail preparation about 17; for cells with trypsin treated P954 tail preparations about .39; for cells with trypsin alone about .50; and for cells with the 300 Kda filtrate about .40.

The results indicate at least a 3 log reduction of P954 titres upon incubation with trypsin under these conditions. Thus, target cell killing activity decreases significantly upon treatment of tail preparations with trypsin.

### E. Host range of P954 tail preparations

To compare the host range of P954 tail preparations to its parent phage, the following comparative experiment was performed. 33 clinical isolates of Staphylococcus aureus (collected from local hospitals from Bangalore, India) were grown in LB overnight, subcultured the next day and lawns prepared on plain LB agar. Suitable amounts of parental P954 phage (10E10 pfu/ml) were spotted on the lawns along with the P954 tail preparations (having approximately 10E2 pfu/ml). The plates were incubated overnight at 30° C and quantitated for lysis.

| Ser # | Isolate | P954T | P954P | Ser # | Isolate | P954T | P954P |
|---|---|---|---|---|---|---|---|
| 1 | B9159 | + | - | 20 | B9198 | - | - |
| 2 | B9160 | + | - | 21 | B9199 | + | - |
| 3 | B9161 | + | - | 22 | B9200 | - | + |
| 4 | B9162 | + | + | 23 | B9201 | + | - |
| 5 | B9163 | + | - | 24 | B9202 | + | - |
| 6 | B9164 | - | - | 25 | B9203 | - | - |
| 7 | B9165 | + | - | 26 | B9204 | + | - |
| 8 | B9166 | + | - | 27 | B9205 | + | - |
| 9 | B9167 | + | - | 28 | B9206 | + | - |
| 10 | B9168 | + | - | 29 | B9207 | + | - |
| 11 | B9169 | - | - | 30 | B9208 | + | - |
| 12 | B9170 | + | - | 31 | B9209 | + | - |
| 13 | B9171 | + | - | 32 | B9210 | - | - |
| 14 | B9172 | + | - | | | | |
| 15 | B9173 | + | - | P954T = P954 tail preparations | | | |
| 16 | B9194 | + | + | P954P = P954 phage | | | |
| 17 | B9195 | + | + | + means lysis seen | | | |
| 18 | B9196 | + | - | - means no lysis | | | |
| 19 | B9197 | + | - | | | | |

These results demonstrate that the P954 phage tail preparations have a broader target killing range compared to the intact parental phage. While the native phage shows only approximately 12% killing across the tested isolates, the tail preparation kills >80% of these same isolates. As suggested above, the killing mechanisms for the tail preparations may be different from intact phage, and the specificity of killing may be somewhat different. But starting with ubiquitous naturally occuring phage, large numbers of isolates may be converted into tail preparations which can be characterized and screened for desirable combinations of properties.

### EXAMPLE 5: Tails from a lytic phage P9042

### A. Size of P9042 killing activity

Generation of P9042 tails was done according to the method described earlier. The P9042 tail preparation was subjected to 300 kDa cutoff membrane and the retentate was used for sample treatment and the assays.

### B. Effect of heat on P9042 phage

The method described above for P954 phage was applied to the P9042 phage. Results indicated that heat inactivates P9042 phage by >99 under the conditions described, though the phage titers decrease by at least 3-4 orders of magnitude upon heat treatment. The tail preparation was also found to be inactivated by heat.

### C. Effect of trypsin on P9042 phage

Likewise, trypsin experiments were performed onthe P9042 lytic phage. A P9042 tail preparation was subjected to 300 Kda cutoff membrane and the retentate used for the killing assay, as described. The A630 for cells alone was about .21; for cells with P9042 phage about .21; for cells with trypsin treated P9042 phage about .23; for cells with P9042 tail preparation about .03; for cells with trypsin treated P9042 tail preparation about .06; for cells with 300 Kda cutoff filtrate about .22; and for cells with heat treated P9042 tail preparation about .21.

Thus, P9042 tails were not inactivated with trypsin and P9042 tail preparation is unstable after heat treatment. The sensitivity of gram positive tails to heat follows the same pattern as their phage types while the trypsin sensitivity is different for different phage tail types. While one of the tested phage tail prepartions is sensitive to trypsin (the P954) the other one (P9042) exhibits resistance to trypsin action under the tested conditions, indicating that they are different from gram negative naturally occurring tails in Pseudomonas (pyocins) which are reported to be trypsin resistant

### EXAMPLE 6: Isolating Prophage Related Constructs

This method describes isolating non-replicating phages which are capable of killing a Staphylococcus target, but is not limited to phages which are directed to such genus of target. Similar methods will be applicable to other target bacteria, e.g., Escherichia, Pseudomonas, Streptococcus, etc. Natural isolates of Staphylococcus are screened for isolates containing one or more lysogenic phage, preferably derived from a tailed phage.

The screening may be performed by many methods. One method is to plate out naturally occurring isolates of target bacteria, e.g., from hospitals or the environment, and screening for low frequency release of the prophage from the bacterial genome, which often occurs upon death of the host cell. The resulting lysogenic phage particles are derived from the prophage.

Upon isolation of the resulting prophage, the host range of the original prophage can be tested on prophage deficient tester strains, e.g., from clinical isolates. Alternatively, the corresponding lysogenic phage released from the strain will exhibit similar killing specificity, but normally the final product should be characterized, e.g., by testing for plaque formation on isolates and characterizing host range specificity.

Having identified the prophage, the sequence may be determined. Critical functions may be deleted by engineering or other methods. Alternatively, functions may be added which prevent replication, e.g., restriction enzymes, etc. Head or other phage structures can be identified and mutagenized, preferably by deletion. Deletion will provide the least likelihood of complementation or spontaneous revertsion. Other methods may be also applied to prevent recombination or reversion, e.g., incorporating other mutations or changes which prevent recombination or complementation. Other critical structures or replication functions may also be targeted, e.g., assembly activities.

Functional prophage may be disrupted by transposon insertion, preferably with a marker such as drug resistance, into the prophage sequence. Since the prophage comprises about 1% of the bacterial genome, about 1% of the transposons incorporated into a genome will be into the prophage sequence. Hybridization, selection, or sequencing methods may be used to determine which transposon integrations are into the prophage. However, if the prophage retains appropriate killing and plaque properties, it likely retains intact tail segments.

Prophage may also be identified using hybridization probes directed to known head protein sequences. With a head gene detected, the gene can be more specifically characterized, and engineering methods may be applied to delete the gene.

Once such killing structures are derived, means to prevent replication in the target may be incorporated. Into the coding segments may be introduced mutations which prevent DNA or phage replication, or functions may be added which can prevent such in target. Other means can be incorporated which require that the structures be made in highly specific host production strains, e.g., with suppressors or complementation.

### EXAMPLE 7: Lytic Phage Derived Constructs

The present invention teaches that tail structures derived from intact phage can be anti-bacterial. Thus, phages accessible from environmental sources serve as starting materials for these killing structures. Plentiful natural phage may be screened for appropriate specificity of target killing. Construction of head deletions, e.g., by mutagenesis, antisense suppression, or engineered, or preparation of tail portions, e.g., by physical separation of tail portions from other phage parts, will provide a non-replication feature. This provides a significant feature for regulatory approval, i.e., that the dose is defined, and/or does not change upon replication after administration.

### EXAMPLE 8: Depletion of Replication Competent Contaminants

Replication competent contaminants may copurify with, but will typically exhibit different properties from, the defined dose compositions described herein. For example, the replication competent contaminants will often contain, or be engineered to express, predicted epitopes absent in the defmed dose compositions. Antibodies to head proteins can be attached to affinity matrix columns for immunoselection or immunoabsorption purify away intact phages. Other affinity methods may be used to deplete them from the preparation, or enzymatic methods, e.g., proteinases, may be used. Engineered susceptible sites on the contaminants should allow means to selectively destroy the undesired contaminants.

In other embodiments, replication competent contaminants will often exhibit different sedimentation properties or size from the defined dose composition. Centrifugation or filtration methods may separate contaminants from the desired compositions.

### EXAMPLE 9: Methods of Determining Anti-bacterial Phage Efficacy and Dosage in animals.

Target bacteria, e.g., P. aeruginosa, is grown in LB medium to an OD600 of 0.2, corresponding to 10E8 CFU/ml. After two rounds of 30 sec of centrifugation at 12000 x g and resuspension in phosphate-buffered saline (PBS), cells are diluted in PBS + 5% mucin to obtain 3x and 10x the minimal lethal dose (MLD) of bacteria per 100 µl. Mice are inoculated intraperitoneally (IP) with 100 µl of bacterial suspension. Controls and anti-bacterial phage dilutions in PBS are injected IP 45 min after infection. Mice receive between 3x10E6 and 3x10E10 killing units of anti-bacterial phage. Mice are allowed to eat and drink ad libitum throughout the 7 day observation period. Those of skill will recognize that dosages for humans can be extrapolated from the mouse dosages.

The following references also describe therapeutic administration of phage: Levin and Bull (1996) American Naturalist 147:881-898; Barrow and Soothill (1997) Trends Microbiol. 5:268-271; Eaton and Bayne-Jones (1934) J. Amer. Med. Assn. 103:1769-1776; 1847-1843; and 1934-1939; Smith and Huggins (1982) J. Gen. Microbiol. 128:307-318; and Smith and Huggins (1983) J. Gen. Microbiol. 129:2659-2675.

Although the foregoing invention has been described in some detail by way of illustration and example for purposes of clarity of understanding, it will be readily apparent to one of ordinary skill in the art in light of the teachings of this invention that certain changes and modifications may be made thereto.

## Claims

1. A composition comprising an anti-bacterial phage-based construct consisting essentially of a phage tail, wherein said phage-based construct is replication incompetent and inhibits growth of target bacteria.

2. A composition according to claim 1, wherein the antibacterial phage-based construct includes less than 10% of the phage heads of the parental phage.

3. A composition according to claim 1 or claim 2, wherein the antibacterial phage-based construct includes less than 1% of the phage heads of the parental phage.

4. A composition according to any of the foregoing claims, wherein the anti-bacterial phage-based construct is from a lytic phage.

5. A composition according to any of the foregoing claims, wherein the anti-bacterial phage-based construct is from a lysogenic phage.

6. A composition according to any of the foregoing claims, wherein the tail is assembled from purified proteins.

7. A composition according to any one of the foregoing claims wherein said anti-bacterial phage-based construct has a different host specificity than the parental phage.

8. A composition according to any one of the foregoing claims further comprising a pharmaceutically acceptable excipient.

9. A composition according to claim 8, further comprising a therapeutic agent selected from the group consisting of an additional replication incompetent anti-bacterial phage, an anti-microbial agent and an antibiotic agent.

10. A method of inhibiting bacterial growth in an ex vivo environment, comprising the step of introducing a composition according to any of claims 1 to 7 to the environment.

11. Use of a composition according to claims 8 or 9 in the manufacture of a medicament for inhibiting a bacterial infection in a host organism.

12. A composition according to claims 8 or 9 for use in a method of inhibiting a bacterial infection in a host organism.

13. The use according to claim 11, or composition for the use of claim 12, wherein the host organism is a human.

14. The use according to claim 11, or composition for the use of claim 12, wherein the host organism is selected from a cow, horse, sheep, pig, dog and cat.

## Patentansprüche

1. Zusammensetzung, die ein antibakterielles, phagenbasiertes Konstrukt umfasst, das im Wesentlichen aus einem Phagenschwanz besteht, worin das phagenbasierte Konstrukt replizierungsunfähig ist und das Wachstum von Zielbakterien hemmt.

2. Zusammensetzung nach Anspruch 1, worin das antibakterielle, phagenbasierte Konstrukt weniger als 10 % der Phagenköpfe des Ausgangsphagen umfasst.

3. Zusammensetzung nach Anspruch 1 oder Anspruch 2, worin das antibakterielle, phagenbasierte Konstrukt weniger als 1 % der Phagenköpfe des Ausgangsphagen umfasst.

4. Zusammensetzung nach einem der vorangegangenen Ansprüche, worin das antibakterielle, phagenbasierte Konstrukt aus einem lytischen Phagen stammt.

5. Zusammensetzung nach einem der vorangegangenen Ansprüche, worin das antibakterielle, phagenbasierte Konstrukt aus einem lysogenen Phagen stammt.

6. Zusammensetzung nach einem der vorangegangenen Ansprüche, worin der Schwanz aus gereinigten Proteinen zusammengesetzt ist.

7. Zusammensetzung nach einem der vorangegangenen Ansprüche, worin das antibakterielle, phagenbasierte Konstrukt eine andere Wirtsspezifität aufweist als der Ausgangsphage.

8. Zusammensetzung nach einem der vorangegangenen Ansprüche, das weiters einen pharmazeutisch annehmbaren Exzipienten umfasst.

9. Zusammensetzung nach Anspruch 8, die weiters ein aus der aus einem weiteren replizierungsunfähigen, antibakteriellen Phagen, einem antimikrobiellen Mittel und einem Antibiotikum bestehenden Gruppe ausgewähltes therapeutisches Mittel umfasst.

10. Verfahren zur Hemmung des Bakterienwachstums in einer *Ex-vivo*-Umgebung, das den Schritt des Einführens einer Zusammensetzung nach einem der Ansprüche 1 bis 7 in die Umgebung umfasst.

11. Verwendung einer Zusammensetzung nach Anspruch 8 oder 9 bei der Herstellung eines Medikaments zur Hemmung einer Bakterieninfektion bei einem Wirtsorganismus.

12. Zusammensetzung nach Anspruch 8 oder 9 zur Verwendung in einem Verfahren zur Hemmung einer Bakterieninfektion bei einem Wirtsorganismus.

13. Verwendung nach Anspruch 11 oder Zusammensetzung zur Verwendung nach Anspruch 12, worin der Wirtsorganismus ein Mensch ist.

14. Verwendung nach Anspruch 11 oder Zusammensetzung zur Verwendung nach Anspruch 12, worin der Wirtsorganismus aus einer Kuh, einem Pferd, einem Schaf, einem Schwein, einem Hund und einer Katze ausgewählt ist.

## Revendications

1. Composition comprenant une construction antibactérienne à base de phage consistant essentiellement en une queue de phage, où ladite construction à base de phase est incompétente pour la réplication et inhibe la croissance de bactéries cibles.

2. Composition selon la revendication 1, dans laquelle la construction antibactérienne à base de phage comprend moins que 10% des têtes de phage du phage parent.

3. Composition selon la revendication 1 ou la revendication 2, dans laquelle la construction antibactérienne à base de phage comprend moins que 1% des têtes de phage du phage parent.

4. Composition selon l'une quelconque des revendications précédentes, dans laquelle la construction antibactérienne à base de phase provient d'un phage lytique.

5. Composition selon l'une quelconque des revendications précédentes, dans laquelle la construction antibactérienne à base de phage provient d'un phage lysogène.

6. Composition selon l'une quelconque des revendications précédentes, dans laquelle la queue est assemblée à partir de protéines purifiées.

7. Composition selon l'une quelconque des revendications précédentes, dans laquelle ladite construction antibactérienne à base de phase a une spécificité d'hôte différente du phage parent.

8. Composition selon l'une quelconque des revendications précédentes, comprenant en outre un excipient acceptable du point de vue pharmaceutique.

9. Composition selon la revendication 8, comprenant en outre un agent thérapeutique sélectionné dans le groupe consistant en un phage additionnel antibactérien incompétent pour la réplication, un agent antimicrobien et un agent antibiotique.

10. Méthode d'inhibition d'une croissance bactérienne dans un environnement ex vivo, comprenant l'étape consistant à introduire une composition selon l'une quelconque des revendications 1 à 7 dans l'environnement.

11. Utilisation d'une composition selon les revendications 8 ou 9, pour la fabrication d'un médicament pour l'inhibition d'une infection bactérienne dans un organisme hôte.

12. Composition selon les revendications 8 ou 9 pour utilisation dans une méthode pour inhiber une infection bactérienne dans un organisme hôte.

13. Utilisation selon la revendication 11, ou composition pour l'utilisation selon la revendication 12, où l'organisme hôte est un humain.

14. Utilisation selon la revendication 11, ou composition pour l'utilisation selon la revendication 12, où l'organisme hôte est sélectionné parmi une vache, un cheval, un mouton, un cochon, un chien et un chat.
